# EUROPEAN PATENT APPLICATION

(11) **EP 4 174 865 A1**
(43) Date of publication of application: **03.05.2023**
(21) Application number: 22204384.6
(22) Date of filing: 28.10.2022
(51) Int. Cl.: G16B 50/00

(54) **CONTROL METHOD AND ANALYSIS SYSTEM**

(30) Priority: 29.10.2021 JP 2021178344
(71) Applicant: SYSMEX CORPORATION, Kobe-shi Hyogo 651-0073 (JP); Riken Genesis Co., Ltd., Tokyo 141-0032 (JP)
(72) Inventor: Wakimoto, Tatsuru, Fukuoka-shi, Fukuoka, 812-0011 (JP); Tanaka, Yoshinori, Tokyo, 141-0032 (JP); Washio, Takanori, Tokyo, 141-0032 (JP)
(74) Representative: Hoffmann Eitle

(57) **Abstract**

A control method of controlling a computer to analyze, at a second facility, nucleic acid sequence data obtained, at a first facility, by a sequencer that reads a nucleic acid sequence, for a gene panel test, comprising receiving, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject; analyzing a first sequence data and a second sequence data corresponding to each of the first library sample and the second library sample linked by the link information; and outputting analysis information based on an analysis result of the first sequence data and an analysis result of the second sequence data, is disclosed.

## Description

### TECHNICAL FIELD

The disclosure relates to a control method of controlling a computer for analyzing nucleic acid sequence data obtained at a first facility using a sequencer that reads a nucleic acid sequence at a second facility in a gene panel test. The invention also relates to an analysis system for analyzing nucleic acid sequence data obtained at a first facility using a sequencer that reads a nucleic acid sequence at a second facility in a gene panel test.

### BACKGROUND ART

With the progress of cancer genome medicine, medical facilities have been developing systems to conduct gene panel tests. Among the medical facilities, the number of medical facilities that install next-generation sequencers (NGS) in their laboratories to accumulate knowledge obtained through gene panel tests to use in the new research is increasing.

On the other hand, data analysis by a bioinformatician is essential in a gene panel test. However, the number of bioinformaticians is small, and it is sometimes difficult to secure human resources. Therefore, there is a need to request an outside specialized organization to analyze nucleic acid sequence data obtained at medical facilities in gene panel tests.

U.S. Patent No. 9,444,880 discloses a system in which a sequencer acquires nucleic acid sequence data, transmits the acquired nucleic acid sequence data to a cloud environment, and analyzes the nucleic acid sequence data by the cloud environment. According to the system disclosed in U.S. Patent No. 9,444,880, nucleic acid sequence data acquired by a sequencer may be analyzed by the cloud environment.

Since NGS usually measures many (e.g., 16 samples) measurement samples (libraries) at the same time, multiple nucleic acid sequence data corresponding to each of the multiple libraries collected from multiple test subjects may be obtained in a single measurement. Furthermore, depending on the type of a gene panel test, it may be necessary to analyze a set of nucleic acid sequence data corresponding to each of the multiple libraries prepared from specimens of the same subject.

For example, in a matched pair test, nucleic acid sequence data of a tumor specimen and nucleic acid sequence data of a non-tumor specimen collected from the same subject are analyzed as a set. In such a case, when the analysis of nucleic acid sequence data in the gene panel test is requested to an outside party, the correct combination of the respective nucleic acid sequence data corresponding to multiple specimens from the same subject must be extracted from the multiple nucleic acid sequence data obtained by NGS at an external analysis facility, and the multiple nucleic acid sequence data of the correct combination must be analyzed.

In a related art such as U.S. Patent No. 9,444,880, it is not considered to extract multiple nucleic acid sequence data of the same subject at an external analysis facility from multiple nucleic acid sequence data obtained by a medical facility, which is the facility requesting analysis, and to perform analysis using multiple nucleic acid sequence data for the subject.

### SUMMARY

A control method and an analysis system according to one or more embodiments may enable accurate and rapid analysis using multiple nucleic acid sequence data of the same subject at a second facility based on nucleic acid sequence data obtained at a first facility.

A control method according to one or more embodiment controls a computer to analyze, at a second facility, nucleic acid sequence data obtained, at a first facility, by a sequencer that reads a nucleic acid sequence, for a gene panel test, may comprise receiving, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject; analyzing a first sequence data and a second sequence data corresponding to each of the first library sample and the second library sample linked by the link information; and outputting analysis information based on an analysis result of the first sequence data and an analysis result of the second sequence data.

An analysis system according to one or more embodiments that analyzes, at a second facility, nucleic acid sequence data obtained, at a first facility, by a sequencer that reads a nucleic acid sequence, for a gene panel test, may comprise: a first computer configured to receive, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject, and send the sequence data set and the link information obtained from the first facility to a second computer; and the second computer configured to analyze a first sequence data and a second sequence data corresponding to each of the first library sample and the second library sample linked by the link information, and output analysis information based on an analysis result of the first sequence data and an analysis result of the second sequence data.

An analysis system according to one or more embodiments that analyzes, at a second facility, nucleic acid sequence data obtained, at a first facility, by a sequencer that reads a nucleic acid sequence, for a gene panel test, may comprise: a computer configured to receive, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject, analyze a first sequence data and a second sequence data corresponding to each of the first library sample and the second library sample linked by the link information, and output analysis information based on an analysis result of the first sequence data and an analysis result of the second sequence data.

A control method and an analysis system according to one or more embodiments may enable the second facility to accurately and quickly perform an analysis using multiple nucleic acid sequence data of the same subject based on nucleic acid sequence data obtained by the first facility.

### BRIEF DESCRIPTION OF DRAWINGS

FIG. 1 is a schematic diagram illustrating a nucleic acid information transmitting and receiving system installed at each facility according to a first embodiment;
FIG. 2 is a flowchart illustrating a process executed by a sequencer;
FIG. 3 is a diagram illustrating an example of a sample sheet;
FIG. 4 is a diagram illustrating an example of sequence run data created by a sequencer;
FIG. 5 is a diagram illustrating a sample sheet of a variation;
FIG. 6A is a diagram illustrating a sample sheet of another variation, and FIG. 6B is a diagram illustrating a sample sheet of yet another variation;
FIG. 7 is a flowchart illustrating processes that each control unit of a data transmitting device, a receiving device, and a nucleic acid sequence analyzer performs;
FIG. 8 is a diagram illustrating an example of a case registration screen displayed on a display unit of a data transmitting device;
FIG. 9 is a flowchart illustrating details of a consistency verification process performed by a control unit of a receiving device;
FIG. 10 is a flowchart illustrating an example of a process procedure when a control unit of a nucleic acid sequence analyzer determines a nucleic acid sequence;
FIG. 11 is a schematic diagram illustrating a generating method of a single mutated reference sequence;
FIG. 12 is a flowchart illustrating a process that a control unit of a nucleic acid sequence analyzer detects a somatic mutation;
FIG. 13 is a flowchart illustrating a process that a control unit of a nucleic acid sequence analyzer detects a germline mutation.
FIG. 14A is a diagram illustrating an example of a nucleic acid sequence of a somatic mutation, and FIG. 14B is a diagram illustrating an example of a nucleic acid sequence of a germline mutation;
FIG. 15 is a diagram illustrating an example of a report format for an analysis report;
FIG. 16 is a flowchart illustrating a process that a sequencer performs in a second embodiment;
FIG. 17 is a diagram illustrating an example of a sample sheet of a second embodiment;
FIG. 18 is a diagram illustrating an example of a case registration screen displayed on a display unit of a data transmitting device in a second embodiment;
FIG. 19 is a flowchart illustrating the details of a consistency verification process that a control unit of a receiving device performs in a second embodiment;
FIG. 20 is a flowchart illustrating a process that a sequencer performs in a third embodiment;
FIG. 21 is a diagram illustrating an example of a sample sheet of a third embodiment;
FIG. 22 is a diagram illustrating an example of a case registration screen displayed on a display unit of a data transmitting device in a third embodiment;
FIG. 23 is a flowchart illustrating details of a consistency verification process that a control unit of a receiving device performs in a third embodiment;
FIG. 24 is a schematic diagram illustrating a nucleic acid information transmitting and receiving system installed at each facility according to a fourth embodiment;
FIG. 25 is a flowchart illustrating processes that each control unit of a data transmitting device and a receiving/analyzing device performs;
FIG. 26 is a flowchart illustrating a process that a sequencer performs in a fifth embodiment;
FIG. 27 is a flowchart illustrating a process that a control unit of a receiving/analyzing device determines a type of mutation of multiple nucleic acid sequence data contained in one sequence data set in a fifth embodiment;
FIG. 28 is a flowchart illustrating processes that each control unit of a data transmitting device, a receiving device, and a nucleic acid sequence analyzer performs in a sixth embodiment; and
FIG. 29 is a flowchart illustrating processes that each control unit of a data transmitting device and a receiving/analyzing device performs in a sixth embodiment.

### DETEILED DESCRIPTION

A control method and an analysis system according to one or more embodiments are described in detail below with reference to the drawings. Embodiments described below are only examples, and the invention is not limited to the following embodiments. Also, in each of the following embodiments, the same symbol is attached to the same configuration in the drawings, and redundant explanations are omitted.

In the following descriptions, tumors can include a benign epithelial tumor, a benign non-epithelial tumor, a malignant epithelial tumor, and a malignant non-epithelial tumor. The origin of the tumor is not restricted. Tumor origins may be exemplified (1) respiratory tissues such as trachea, bronchus, or lungs; (2) gastrointestinal tissues such as nasopharynx, esophagus, stomach, duodenum, jejunum, ileum, cecum, appendix, ascending colon, transverse colon, sigmoid colon, rectum, or anal region; (3) liver; (4) pancreas; (5) urinary system tissues such as bladder, ureter, or kidney; (6) female reproductive system tissues such as ovaries, fallopian tubes, and uterus; (7) mammary gland; (8) male reproductive system tissues such as prostate gland; (9) skin; (10) endocrine system tissues such as hypothalamus, pituitary gland, thyroid gland, parathyroid gland, and adrenal gland; (11) central nervous system tissue; (12) bone and soft tissues; (13) hematopoietic tissues such as bone marrow and lymph nodes; (14) blood vessels, etc.

In the following descriptions, a sample is a sample prepared from a specimen such as tissue, body fluid, and excrement collected from a subject, and includes nucleic acids derived from tumor cells or non-tumor cells. Nucleic acids include deoxyribonucleic acid (hereinafter referred to as DNA) or ribonucleic acid (hereinafter referred to as RNA). Nucleic acids may be present intracellularly or may be present in body fluids by leaking out of a cell when the cell is destroyed or dies. Nucleic acids present in body fluids include, for example, cell free DNA (cfDNA) and circulating tumor DNA (ctDNA). Body fluids are, for example, blood, bone marrow fluid, ascites, pleural fluid, and spinal fluid. Excretions are, for example, stool, urine, and sputum. Fluids obtained after washing a part of a patient's body, such as intra-abdominal lavage fluid or colonic lavage fluid, may be used as a specimen. The amount of nucleic acid contained in the specimen is not limited as long as a nucleic acid sequence may be detected. Also, when obtaining nucleic acid sequence data derived from non-tumor cells, a specimen containing nucleic acid derived from non-tumor cells is used. The concentration of non-tumor cells in the above-mentioned tissues, body fluids, etc. is not limited as long as the sequence of nucleic acid present in the non-tumor cells may be detected. Here, when tumor cells are derived from solid tumors, for example, peripheral blood, oral mucosal tissues, skin tissues, etc. may be used as a specimen containing nucleic acid derived from non-tumor cells. When tumor cells are derived from the hematopoietic tissue, for example, oral mucosal tissue, skin tissue, etc. may be used as a specimen containing nucleic acid derived from non-tumor cells.

A specimen may be collected from a fresh tissue, fresh-frozen tissue, paraffinembedded tissue, etc. Collecting a specimen may be made according to a known method. Also, in the following descriptions, when a sample containing nucleic acid derived from tumor cells and a sample containing nucleic acid derived from non-tumor cells are collected from the same subject, the sample containing nucleic acid derived from non-tumor cells and the sample containing nucleic acid derived from tumor cells may be collected at the same time or at different times.

A gene to be analyzed for a nucleic acid sequence is not limited as long as the gene is a gene that exists on the human genome. Preferably, the gene may be a gene which is associated with tumor onset, prognosis, and therapeutic efficacy. Also, in the following descriptions, a gene mutation may be a disease-related mutation or a sequence polymorphism of a gene. A gene "polymorphism" includes a SNV (Single Nucleotide Variant, single nucleotide polymorphism), a VNTR (Variable Nucleotide of Tandem Repeat, repetitive sequence polymorphism), a STRP (Short Tandem Repeat Polymorphism), and a microsatellite polymorphism. Also, a genetic mutation may be a fusion mutation.

In the following description, nucleic acid sequence data is not limited as long as the data reflects a nucleic acid sequence. Information on a genetic mutation is not limited as long as it is information on a genetic mutation possessed by a subject from whom a specimen is collected. For example, the information about a genetic mutation can include at least a label indicating the name of the gene in which the mutation is detected. Preferably, the information on a genetic mutation may include a label indicating the name of the gene in which a mutation is detected and information on the detected nucleic acid sequence and/or an amino acid sequence produced by the mutation. Also, the information on a gene mutation may include locus information of the gene in which the mutation is detected, reference sequence information, and information on a mutated sequence possessed by the subject. Furthermore, the information on a gene mutation is not limited to information that detects the presence or absence of a mutation, and, for example, may be information that suggests a possibility of the presence of a gene mutation (e.g., mosaic mutation).

### First Embodiment

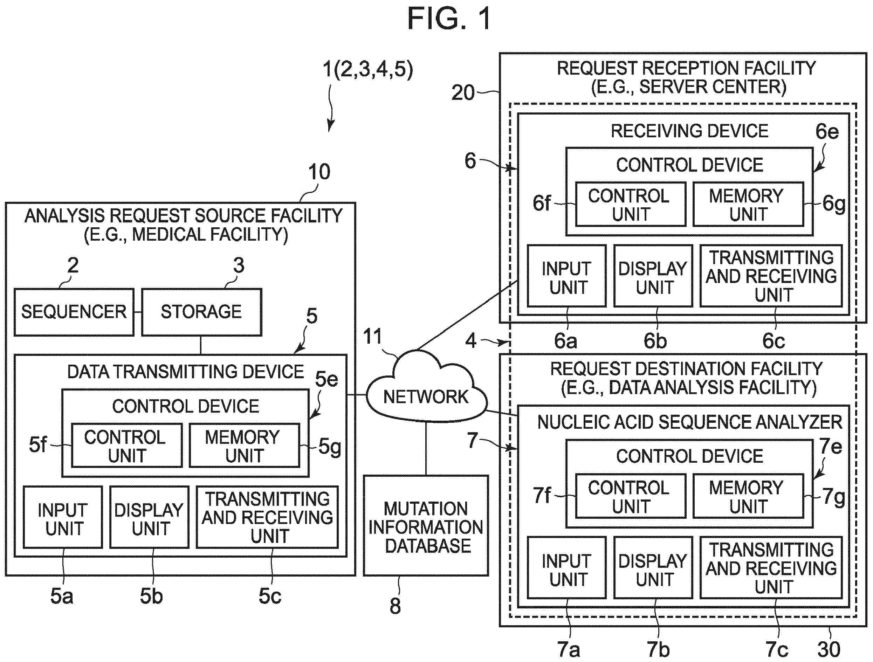

FIG. 1 is a schematic diagram of a nucleic acid information transmitting and receiving system 1 installed at each facility according to a first embodiment or embodiments. First, using FIG. 1, a schematic configuration of the nucleic acid information transmitting and receiving system 1 and an outline of the main information flow in the nucleic acid information transmitting and receiving system 1 are explained. The nucleic acid information transmitting and receiving system 1 is equipped with a sequencer 2, a storage (storage device) 3, a data transmitting device 5, and an analysis system 4, and the analysis system 4 has a receiving device 6 and a nucleic acid sequence analyzer 7. The data transmitting device 5, the receiving device 6, and the nucleic acid sequence analyzer 7 are connected to each other via a network 11, which is the internet. The network 11 is further connected to a mutation information database 8.

The sequencer 2, the storage 3, and the data transmitting device 5 are installed in an analysis request source facility 10, for example, a hospital (medical facility), a testing center, or a biomedical science laboratory. The sequencer 2 is a next-generation sequencer (NGS). Hereafter, when referring to a sequencer, it means a next-generation sequencer. The sequencer 2 is a device that reads base sequence information of nucleic acid, for example, an MiSeq system (manufactured by Illumina, Inc.), a NextSeq550 system (manufactured by Illumina, Inc.), an Ion Gene Studio S5 system (manufactured by Thermo Fisher Scientific, Inc.), an Ion Torrent Genexus system (manufactured by Thermo Fisher Scientific, Inc.), etc. may be used. The sequencer 2 reads a nucleic acid sequence of multiple library samples (e.g., 16 samples) in one sequence run. The sequencer 2 reads nucleic acid sequences from each of the multiple library samples including a first library sample and a second library sample prepared from specimens collected from the same subject in one sequence run and generates a sequence data set containing multiple nucleic acid sequences corresponding to each library sample. The sequencer 2 may generate a sequence data set corresponding to one subject or may generate multiple sequence data sets corresponding to each of the multiple subjects in one sequence run. In addition, the sequencer 2 is inputted with link information indicating that the first library sample and the second library sample are prepared from the specimen of the same subject. A library sample is a sample prepared for reading a nucleic acid sequence, also called a library. A library sample may be prepared using Onco Guide NCC Onco Panel Kit (manufactured by Sysmex Corporation), for example. The link information is information that indicates that multiple library samples are prepared from the specimen of the same subject. The link information may include sample identification information to identify the first library sample and the second library sample, and/or subject identification information to identify the same subject that is the collection source of the specimens corresponding to the first library sample and the second library sample.

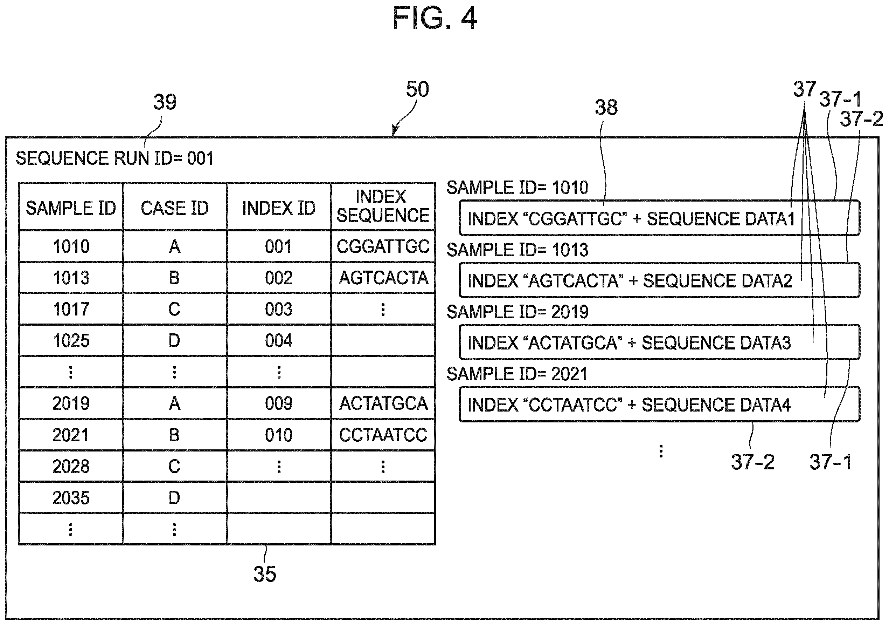

The sequencer 2, based on the generated sequence data set and the link information, generates sequence run data including the sequence data set and the link information, and stores them in the storage 3. The sequence data set and the sequence run data are described in detail below using FIG. 4. The storage 3 is a Network Attached Storage (NAS). NAS is configured as a storage device that may be directly connected to a network.

The data transmitting device 5 may be a computer. The data transmitting device 5 is equipped with an input unit 5a, a display unit 5b, a transmitting/receiving unit 5c, and a control device 5e, and the control device 5e includes a control unit 5f and a memory unit 5g. The input unit 5a is used to input data and consists of a keyboard and a mouse. The display unit 5b consists of a liquid crystal panel and displays an image. The display unit 5b may consist of an organic EL panel. The input unit 5a and the display unit 5b may consist of a touch panel that integrates a touch sensor and a display. The transmitting/receiving unit 5c is an interface, which may include a hardware interface such as a transceiver or transceivers, or individual transmitters and receiver circuits, for transmitting and receiving data to and from an external device via the network 11 connected to the data transmitting device 5, and, for example, consists of an interface compatible with Ethernet. The control unit 5f is a CPU, and the memory unit 5g consists of SSD and semiconductor memory.

The data transmitting device 5 reads the sequence run data from the storage 3 via the transmitting/receiving unit 5c and transmits the sequence run data to the receiving device 6 via the transmitting/receiving unit 5c and the network 11.

The receiving device 6 is installed at a request reception facility 20, e.g., a server center. The analysis request source facility 10 and the request reception facility 20 are different facilities. The receiving device 6 may be a computer that constitutes a cloud system. The server center may be a facility of a cloud service provider or a facility of a company that provides a nucleic acid sequence analysis service. The receiving device 6 is a computer. The receiving device 6 has an input unit 6a, a display unit 6b, a transmitting/receiving unit 6c, and a control device 6e. The control device 6e includes a control unit 6f and a memory unit 6g. Hardware configurations of the input unit 6a, the display unit 6b, the transmitting/receiving unit 6c, and the control device 6e are the same as those of the input unit 5a, the display unit 5b, the transmitting/receiving unit 5c, and the control device 5e, respectively. The receiving device 6 transmits sequence run data to the nucleic acid sequence analyzer 7 via the transmitting/receiving unit 6c and the network 11.

The nucleic acid sequence analyzer 7 is installed at a request destination facility 30, e.g., a data analysis facility. The analysis request source facility 10 and the request destination facility 30 are different facilities. The request reception facility 20 and the request destination facility 30 are different facilities, but they may be the same facility. The nucleic acid sequence analyzer 7 may be a computer that constitutes a cloud system. The data analysis facility may be a facility of a cloud service provider or a facility of a company that provides a nucleic acid sequence analysis service. The nucleic acid sequence analyzer 7 is a computer. The nucleic acid sequence analyzer 7 has an input unit 7a, a display unit 7b, a transmitting/receiving unit 7c, and a control device 7e. The control device 7e includes a control unit 7f and a memory unit 7g. Hardware configurations of the input unit 7a, the display unit 7b, the transmitting/receiving unit 7c, and the control device 7e are the same as those of the input unit 5a, the display unit 5b, the transmitting/receiving unit 5c, and the control device 5e, respectively. The nucleic acid sequence analyzer 7 can access the mutation information database 8 via the network 11.

The mutation information database 8 consists, for example, of an external public sequence information database or a public known mutation information database. The control device 7e of the nucleic acid sequence analyzer 7 checks each of the nucleic acid sequence data included in the sequence run data received from the receiving unit 6 with reference nucleic acid sequence data stored in the mutation information database 8 and generates mutation information of genes for each of the nucleic acid sequence data.

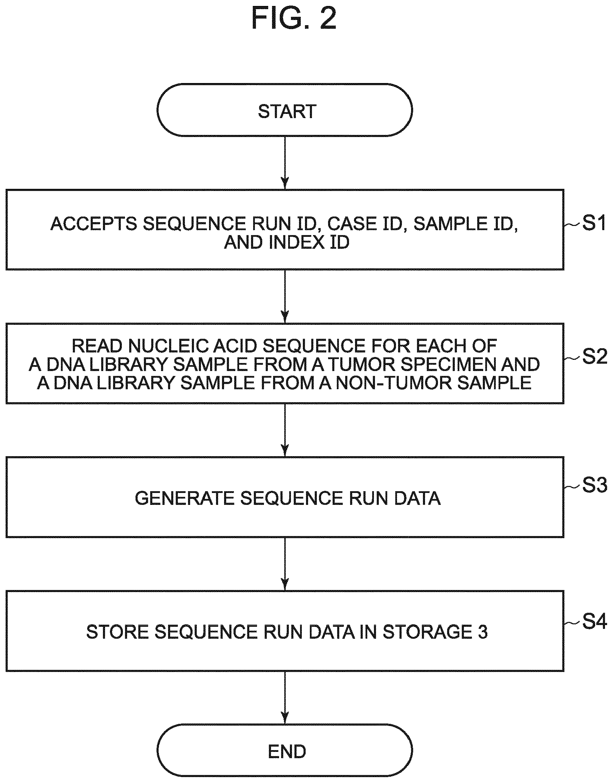

FIG. 2 is a flowchart illustrating a process executed by the sequencer 2. Referring to FIG. 2, the process executed by the sequencer 2 is explained. First, in step S1, the sequencer 2 accepts a sequence run ID, a case ID, a sample ID, and an index ID and generates a sample sheet, which is an electronic file. The sequence run ID is information that identifies sequence run data. The sample sheet includes the case ID, the sample ID, and the index ID. One sample sheet is generated per sequence run, i.e., per cartridge. Sequences of multiple library samples (e.g., 16 samples) are read per sequence run, i.e., per cartridge. Multiple library samples are prepared by pretreating each of the multiple samples (e.g., 16 samples) prepared from each tumor tissue and non-tumor tissue (e.g., blood) from multiple subjects (e.g., 8 subjects) with a reagent and by adding different index sequences to the multiple samples. In a first embodiment, each library sample is a sample prepared from DNA. The case ID is information that identifies a subject from whom each library sample is taken. The sample ID is information that identifies each library sample. The index ID is information that identifies an index sequence attached to each library sample.

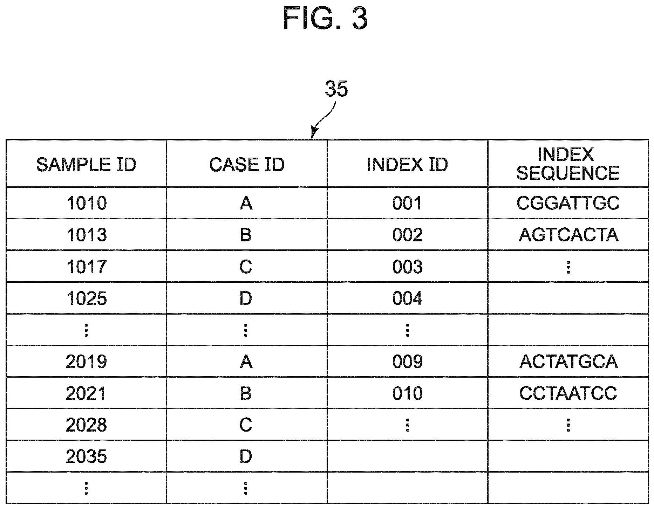

FIG. 3 is a diagram illustrating an example of a sample sheet 35. In the example illustrated in FIG. 3, a sample ID is associated with a case ID. Library samples with the same case IDs are samples prepared from specimens of the same subject. The sample ID on the sample sheet 35 is an example of sample identification information, and the case ID is an example of subject identification information and an example of link information indicating that multiple library samples are prepared from the specimen of the same subject. Each sample ID is further associated with an index ID and an index sequence. The index sequence is information indicating an index sequence added to the library sample.

For example, a library sample with a sample ID 1010 is prepared from a specimen of a subject A who has a specific disease and is a sample with an index ID of 001. The index sequence of the library sample is CGGATTGC. By determining that the partial nucleic acid sequence of CGGATTGC is included in a nucleic acid sequence read by the sequencer 2, the nucleic acid sequence data may be identified as the nucleic acid sequence data of the library sample with the sample ID of 1010. A library sample with a sample ID 2019 is prepared from a specimen of the subject A who has a specific disease and is a sample with an index ID of 009. The index sequence of the prepared library sample is ACTATGCA. The library sample with the sample ID 1010 and the library sample with the sample ID 2019 have the same case ID (A) so that it indicates that both library samples are prepared from the specimen of the same subject A. Similarly, the library sample with a sample ID 1013 and the library sample with a sample ID 2021 have the same case ID (B) so that it indicates that both library samples are prepared from the specimen of another identical subject B. Furthermore, in a first embodiment, when a sample ID is an ID starting from 1, it indicates that the corresponding library sample is derived from a tumor cell, and when a sample ID is an ID starting from 2, it indicates that the corresponding library sample is derived from a non-tumor cell. Thus, by referring to the data on the sample sheet, multiple library samples derived from the same subject may be identified, as well as information on whether each library sample is derived from a tumor cell or a non-tumor cell.

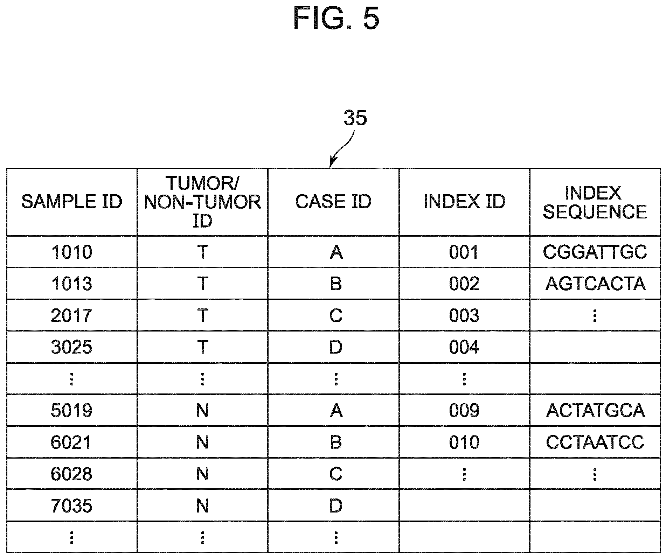

The sample sheet may be any notation that includes the link information. For example, as illustrated in FIG. 5, i.e., a figure illustrating a variation of sample sheet 35', a tumor/non-tumor ID column may be added to the sample sheet 35 to identify whether each library sample is derived from a tumor specimen or a non-tumor specimen. In the sample sheet 35', T indicates that the sample is derived from a tumor specimen, and N indicates that the sample is derived from a non-tumor specimen. Accordingly, it is no longer necessary to indicate whether the corresponding library sample is of tumor cell origin or non-tumor cell origin by the sample ID, and assignment of sample IDs may be easily done.

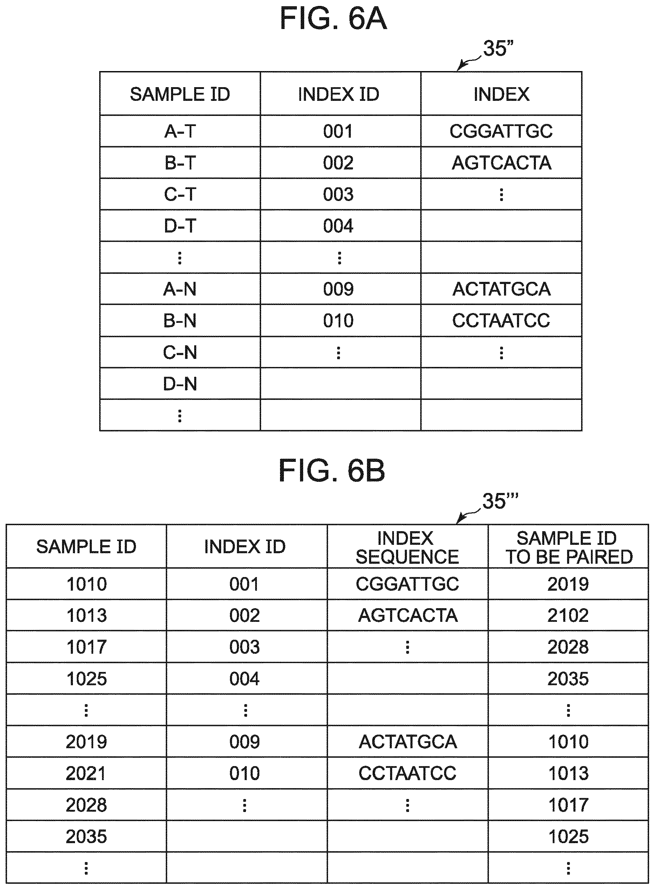

Also, the notation of the sample sheet, for example, as illustrated in FIG. 6A, i.e., a figure illustrating a variation of sample sheet 35", a case ID column may be omitted. Accordingly, the sample ID may consist of two pairs of letters, symbols or numbers. Then, for example, the first letter, symbol or number may indicate identification information that can identify the subject, and the second letter, symbol or number may indicate identification information that can identify a tumor specimen or a non-tumor specimen. For example, in the table of the sample sheet 35", the first capital letters of the alphabets, "A", "B", "C", and "D" may be identification information that identify the subject from whom the specimen is taken, and in the second capital letter of the alphabet, "T" or "N", "T" may be identification information that indicates a tumor specimen, and "N" may be identification information that indicates a non-tumor specimen. In the sample sheet 35", the sample ID indicates that multiple library samples are prepared from the specimen of the same subject. For example, a library sample with a sample ID A-T and a library sample with a sample ID A-N are indicated to be both samples prepared from the specimen of the subject A. Therefore, the sample ID on the sample sheet 35" is an example of the link information indicating that multiple library samples are prepared from the specimen of the same subject. In the examples described using the above-mentioned FIG. 3, FIG. 5, and FIG. 6A, the link information is the sample ID or the case ID. Therefore, the link information indicates that multiple library samples are prepared from the specimen of the same subject and identifies the library sample or the subject. In other words, in the examples described using the above-mentioned FIG. 3, FIG. 5, and FIG. 6A, the information that identifies the sample or the subject is also used as the link information, and there is no need to separately input the link information into the sequencer 2.

FIG. 6B illustrates another variation of the sample sheet. As illustrated in FIG. 6B, a sample sheet 35"', compared to the sample sheet 35, does not contain a case ID column and includes a column of paired sample IDs. The paired sample IDs are sample IDs that identify library samples prepared from specimens of the same subject as the subject from whom the library sample specimens identified by the corresponding sample IDs are collected. For example, for a library sample with a sample ID of 1010, the paired sample ID is 2019 so that it may be identified that the library sample with the sample ID of 2019 is prepared from the specimen of the same subject. Therefore, the paired sample ID is an example of link information. In the present example, in step S1 (see FIG. 2), instead of the case ID, the paired sample ID is input to the sequencer 2. Accordingly, when information that serves as link information is input to the sequencer 2 separately from the information that identifies the sample or the subject, it is not necessary to input the information that identifies the sample or the subject. Also, in the sample sheet 35"', the sample IDs that are paired are input to both the library sample prepared from the tumor specimen and the library sample prepared from the non-tumor specimen, but one of the two may be omitted. In addition, the notation on the sample sheet may be a notation with link information added to the known notation used in the sequencer 2. The notation of the sample sheet may be any notation as long as the notation can recognize information that can identify the corresponding subject for each library sample and information that can identify whether the library sample is derived from a tumor cell or a non-tumor cell.

Referring again to FIG. 2, next, a process that the sequencer 2 performs next is explained. A user of the sequencer 2 dispenses multiple pre-prepared library samples (e.g., 16 samples) into each well of a single cartridge, sets the cartridge in the sequencer 2, and instructs the sequencer 2 to start sequence reading. When the start of sequence reading is instructed by the user, the sequencer 2 reads nucleic acid sequences for each of the multiple library samples in step S2. In a first embodiment or embodiments, the sequencer 2 reads a library sample of DNA prepared from a tumor specimen and a library sample of DNA prepared from a non-tumor specimen for each of the multiple subjects. Then, the sequencer 2 generates sequence run data in step S3. Then, in the next step S4, the generated sequence run data is stored in the storage 3, and the process is completed.

FIG. 4 is a diagram illustrating an example of a sequence run data 50 created by the sequencer 2. The sequence run data 50 is an electronic folder that contains electronic files, and a sequence run ID 39 accepted in step S1 is assigned as the folder name. The sequence run data 50 contains the sample sheet 35 and nucleic acid sequence data 37 read from each library sample in step S2. The sequencer 2 compares an index sequence 38 contained in each of the nucleic acid sequence data 37 with each index sequence contained in the sample sheet 35 and associates the sample IDs having the same index sequences to each other with the nucleic acid sequence data 37. Also, the plurality of nucleic acid sequence data 37 corresponding to each of the plurality of sample IDs corresponding to the same case IDs constitute one sequence data set. In the example in FIG. 4, two of the nucleic acid sequence data 37 corresponding to each of the library samples with the sample ID 1010 and the library sample with the sample ID 2019 corresponding to the case A constitute a sequence data set 37-1, and two of the nucleic acid sequence data 37 corresponding to each of the library samples with a sample ID 1013 and the library sample with a sample ID 2021 corresponding to the case B constitute a sequence data set 37-2. The number of sequence data sets included in one sequence run, i.e., 1 sequence run data 50, is not particularly limited, but from the viewpoint of reading more nucleic acid sequences of a subject in one sequence run, it is preferred to be five or more.

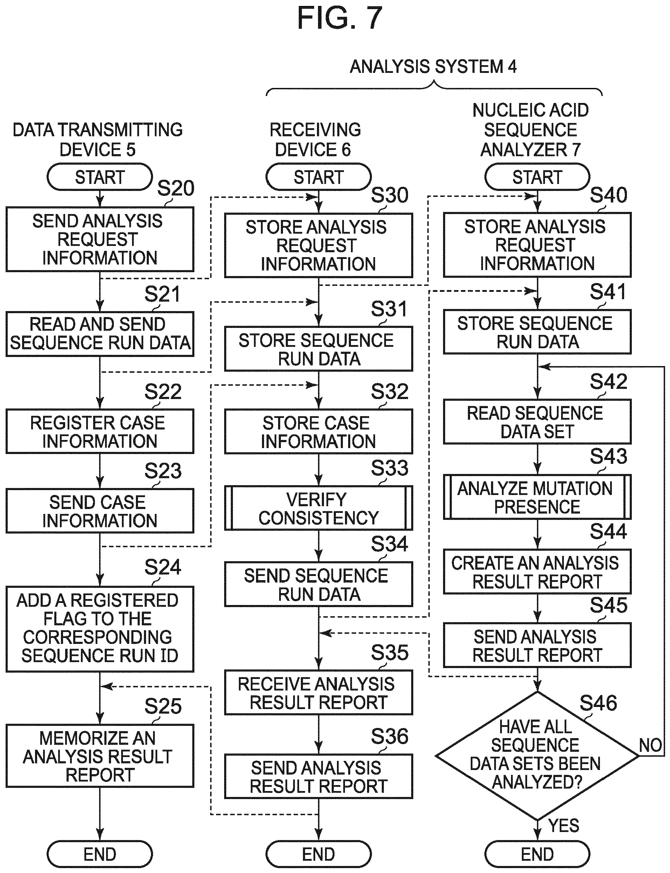

FIG. 7 is a flowchart illustrating processes executed by each control unit of the data transmitting device 5, the receiving device 6, and the nucleic acid sequence analyzer 7. Referring to FIG. 7, first, a process executed by the control unit 5f of the data transmitting device 5 is described. When the control unit 5f receives an analysis instruction for sequence run data stored in the storage 3 from a user of the data transmitting device 5, the analysis request information is sent to the receiving device 6 in step S20. The analysis request information is information that the user inputs by operating the input unit 5a and includes the case information of the subject, the type of a gene panel test, and the information of the request source facility 10. The case information of the subject includes a case ID. The information of the request source facility 10 includes identification information to identify the name of the request source facility and the request source facility. In addition, the analysis request information may include at least one of the case information of the subject, the type of the gene panel test, and the information of the request source facility 10. Also, for example, when there is an agreement between the request source facility 10 and the request reception facility 20 that the transmission of sequence run data or case information is considered as an analysis request, the process of step S20 may be omitted.

In step S21, the control unit 5f reads the sequence run data from the storage 3 and sends it to the receiving device 6. In step S22, the control unit 5f makes the display unit 5b display a case registration screen and accepts registration of the case information. The case information includes input information indicating that the library sample derived from the tumor specimen and the library sample derived from the non-tumor specimen are prepared from the same subject. Specifically, the input information includes a sample ID for the tumor specimen-derived library sample, a sample ID for the non-tumor specimen-derived library sample, and 1 case ID corresponding to both sample IDs.

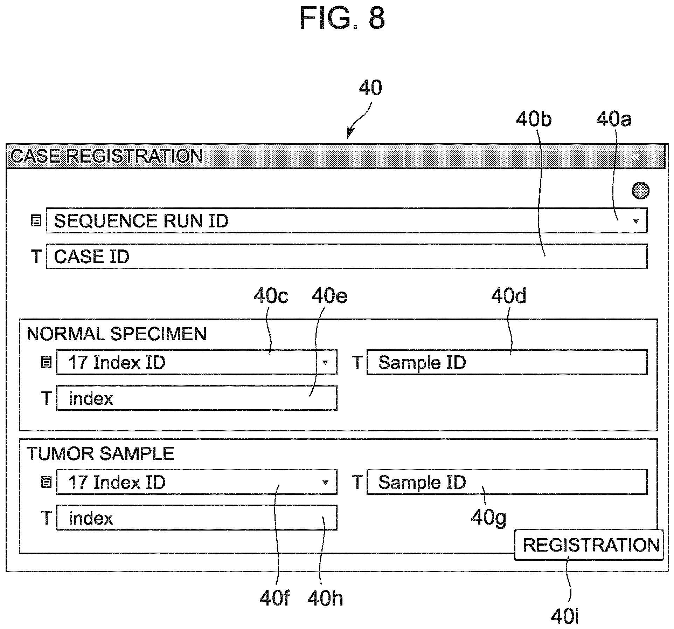

FIG. 8 is a diagram illustrating an example of a case registration screen 40 displayed on the display unit 5b. As illustrated in FIG. 8, the case registration screen 40 displays a registration unit 40a that registers a sequence run ID, a registration unit 40b that registers a case ID, for a library sample derived from a normal specimen (non-tumor specimen), a registration unit 40c that registers an index ID, a registration unit 40d that registers a sample ID, and a registration unit 40e that registers an index sequence, for a library sample derived from a tumor specimen, a registration unit 40f that registers an index ID, a registration unit 40g that registers a sample ID, and a registration unit 40h that registers an index sequence, and a registration button 40i. The registration unit 40a, the registration unit 40c, and the registration unit 40f consist of pull-down list formats, and when the pull-down list is expanded, among the sequence run data that the control unit 5f reads from storage 3, the sequence run ID and the index ID included in the sequence run data, to which a registered flag described below is not added, are displayed in the list. The registration unit 40b, the registration unit 40d, the registration unit 40e, the registration unit 40g, and the registration unit 40h are configured so that a user can operate the keyboard to input numerical values, letters, or symbols. The user of the data transmitting device 5 operates the input unit 5a to input information into the registration units 40a to 40h for each subject, and when the registration of 1 subject is completed, the user selects the registration button 40i. The user of the data transmitting device 5 repeats an operation that inputs information into the registration units 40a to 40h for each subject and selects the registration button 40i until input of all case IDs included in 1 sequence run is completed.

The registration units 40a to 40h may be configured in pull-down list formats, except for the registration unit 40a, the registration unit 40c, and the registration unit 40f, and the registration unit 40a, the registration unit 40c, and the registration unit 40f may be configured so that numerical values, etc., are entered. In addition, the registration unit 40e and the registration unit 40h may be configured so that the corresponding index sequence is read from the sequence run data when an index ID is input to the registration unit 40c or the registration unit 40f, and the corresponding index sequence is displayed in the registration unit 40e or the registration unit 40h. As a case registration screen, any screen may be employed as long as the screen can register information that can identify the corresponding same subject for each library sample of a normal specimen (non-tumor specimen) and a tumor specimen.

Referring again to FIG. 7, a process that the control unit 5f of the data transmitting 5 executes next is described. When the process of step S22 is completed, i.e., input of all case IDs contained in 1 sequence run data is completed and the registration button 40i is selected, the control unit 5f sends the case information entered into the registration units 40a to 40h in step S22 to the receiving device 6 in step S23. As described above, the case information includes input information indicating that the library samples derived from the tumor specimen and the library sample derived from the non-tumor specimen are prepared from the same subject. In a first embodiment, the input information is the sample ID of the normal specimen entered via the registration unit 40d, the sample ID of the tumor specimen entered via the registration unit 40g, and a case ID entered via the registration unit 40b. The sample ID is information that identifies each library sample, and the case ID is information that identifies the subject. In step S24, the control unit 5f adds a flag indicating that the sequence run data is registered to the sequence run ID corresponding to the sequence run data transmitted to the receiving device 6 in step S21.

Next, a process that the control unit 6f of the receiving device 6 performs is described. When analysis request information from the data transmitting device 5 is sent, the control unit 6f receives the analysis request information and stores it in the memory unit 6g in step S30. When sequence run data is sent from the data transmitting device 5, the control unit 6f receives the sequence run data and stores it in the memory unit 6g in step S31. Also, when the case information from the data transmitting device 5 is sent, the control unit 6f receives the case information and stores it in the memory unit 6g in step S32. In the subsequent step S33, the control unit 6f conducts verification of consistency and determines whether the link information contained in the sequence run data stored in step S31 is consistent with the input information contained in the case information stored in step S32.

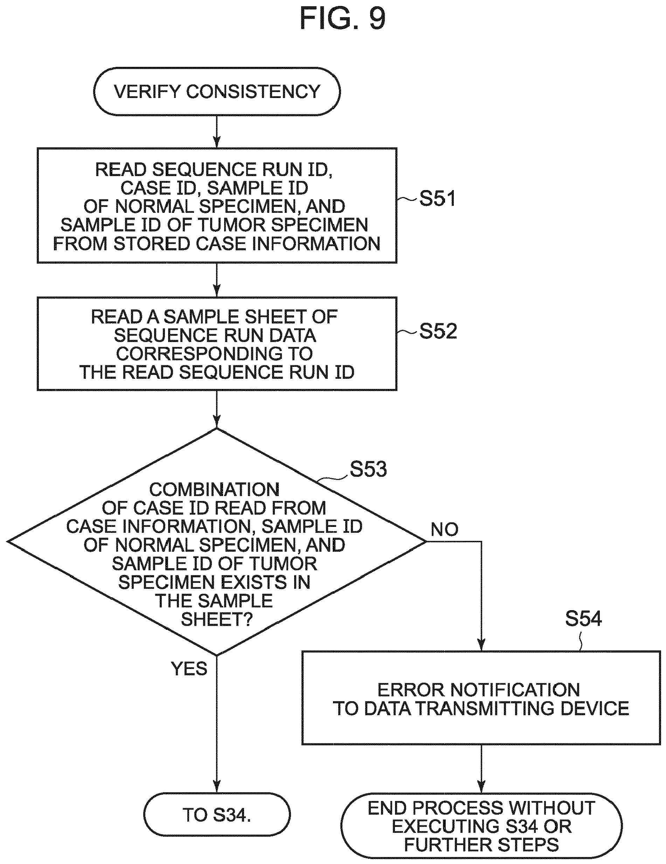

FIG. 9 is a flowchart illustrating the details of the consistency verification process performed by the control unit 6f in step S33. In step S51, the control unit 6f reads a sequence run ID, a case ID, a sample ID of a normal specimen, and a sample ID of a tumor specimen from the stored case information. The sequence run ID is the information entered via the registration unit 40a of the case registration screen 40 (see FIG. 8), the case ID is the information entered via the registration unit 40b, the sample ID of a normal specimen is the information entered via the registration unit 40d, and the sample ID of a tumor specimen is the information entered via the registration unit 40g. In step S52, the control unit 6f reads the information listed on the sample sheet of the sequence run data that is assigned the same sequence run ID as the sequence run ID read in step S52. Then, in step S53, the control unit 6f determines whether or not the combination of the case ID, the sample ID of the normal specimen, and the sample ID of the tumor specimen read from the case information in step S51 exists on the sample sheet.

When the control unit 6f makes a negative judgment in step S53 (in the case of "No"), the control unit 6f performs an error notification indicating that the link information of the sequence run data and the case information are inconsistent to the data transmitting device 5 in step S54 and terminates the process without executing step S34 and thereafter (see FIG. 7). The control unit 5f of the data transmitting device 5 that receives the error notification outputs the error information indicating the link information of the sequence run data and the case information are inconsistent to the display unit 5b. The output of the error information allows the user of the data transmitting device 5 to recognize that an error exists in at least one of the information on the sample sheet and the manually entered case information. On the other hand, when the control unit 6f of the receiving device 6 makes a positive judgment in step S53 (in the case of "Yes"), the control unit 6f returns the process to step S34 (see FIG. 7).

According to a first embodiment or embodiments, in step S53, it is determined whether the link information of the sequence run data and the case information are consistent or not, and if these two pieces of information are not consistent, the process is terminated without moving to the next step. Therefore, in each subject, the nucleic acid sequence data derived from the tumor specimen and the nucleic acid sequence data derived from the non-tumor specimen may be accurately linked to the subject. Thus, even in the case of a matched pair test, in which multiple nucleic acid sequence data derived from the same subject are analyzed, incorrect analysis by mistaking the nucleic acid sequence data may be surely prevented.

Referring again to FIG. 7, a process that the control unit 6f of the receiving device 6 performs next is described. When an affirmative decision is made in step S53 (see FIG. 9), the control unit 6f sends the sequence run data stored in step S31 to the nucleic acid sequence analyzer 7 in step S34. Next, a process that the control unit 7f of the nucleic acid sequence analyzer 7 performs is described. In step S40, the control unit 7f receives the analysis request information sent from the receiving device 6 and stores it in the storage unit 7g. In step S41, the control unit 7f receives the sequence run data sent from the receiving device 6 and stores it in the memory unit 7g. In step S42, the control unit 7f reads 1 sequence data set from the stored sequence run data. As mentioned above, the sequence data set contains multiple nucleic acid sequence data 37 corresponding to the same case ID; therefore, the control unit 7f can extract multiple nucleic acid sequence data 37 corresponding to the same case ID as 1 sequence data set, using the case ID that is the link information as a search key.

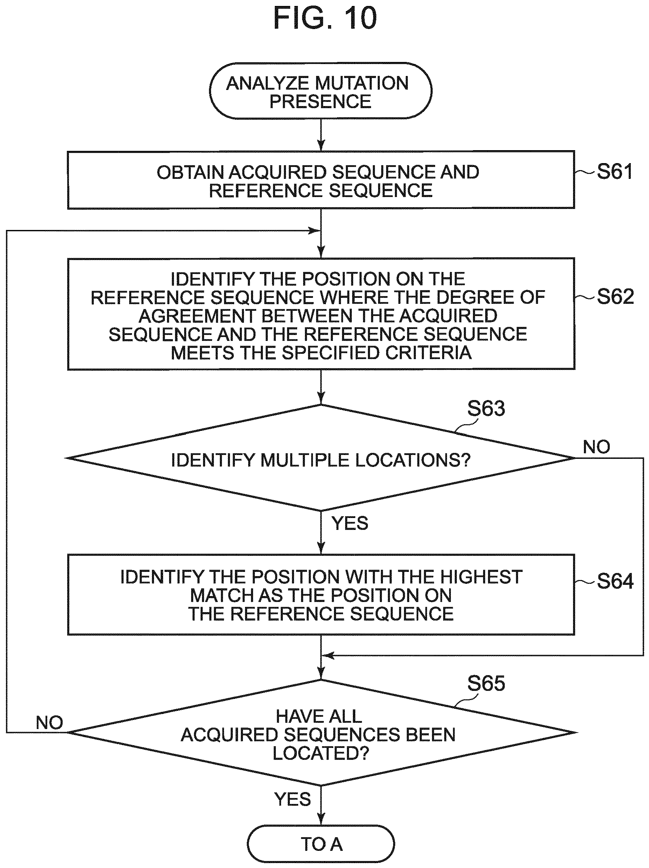

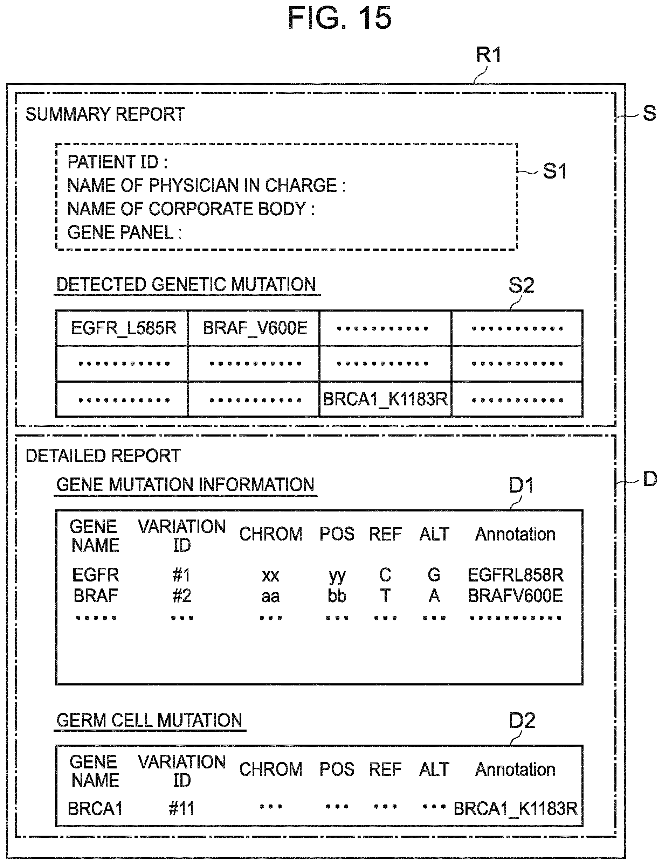

In step S43, the control unit 7f analyzes the presence or absence of a mutation using the information on nucleic acid sequences of tumor cells in the mutation information database 8 for each nucleic acid sequence data in the sequence data set extracted in step S42. In step S44, the control unit 7f creates an analysis result report based on the presence or absence of the mutation. In step S45, the control unit 7f sends the analysis result report to the receiving device 6. The process of step S43 is described in detail below using FIGS 10 to 14. The analysis result report is described in detail below using FIG. 15. In step S46, the control unit 7f determines whether all sequence data sets included in the sequence run data stored in step S41 are extracted. When all sequence data sets are extracted (in the case of "Yes"), the control unit 7f terminates the process, and when all sequence data sets are not extracted (in the case of "No"), the control unit 7f returns the process to step S42 and performs the process of steps S42 to S46 again.

Meanwhile, the control unit 6f of the receiving device 6 receives the analysis result report in step S35, sends the analysis result report to the data transmitting device 5 in step S36, and terminates the process. The control unit 5f of the data transmitting device 5 receives the analysis result report, stores it in the memory unit 5g in step S25, and terminates the process, allowing the physician in charge of the subject to display and view the analysis report stored in the storage unit 5g on the display unit 5b at any time.

Next, referring to FIG. 10, the process of step S43 by the control unit 7f is described in detail. FIG. 10 is a flowchart illustrating an example of the process procedure when the control unit 7f of the nucleic acid sequence analyzer 7 determines the nucleic acid sequence. In step S61, the control unit 7f acquires 1 nucleic acid sequence data 37 (hereinafter referred to as "acquired sequence") from the data set extracted in step S42. Also, the control unit 7f downloads a reference sequence from the mutation information database 8 and stores it in the storage unit 7g.

A reference sequence is a sequence to which the acquired sequence is mapped in order to determine which region on the gene the acquired sequence corresponds to and which mutation on the gene the acquired sequence corresponds to. For each gene to be analyzed, (1) a wild-type reference sequence, which is a partial or complete sequence of a wild-type exon, may be used as a reference sequence. Also, (2) a single mutated reference sequence, which is a rearranged sequence containing known polymorphisms and mutations linked from the wild-type exon sequence, may be used as a reference sequence. A single mutation reference sequence is a sequence generated by linking two or more rearranged sequences related to the gene to be analyzed into a single link for each gene to be analyzed. The single mutation reference sequence is used as a mutation reference sequence including the rearranged sequence when mapping the acquired sequence. In addition, instead of a single mutation reference sequence consisting of two or more rearranged sequences linked together, two or more unconnected rearranged sequences may be used as the mutation reference sequence.

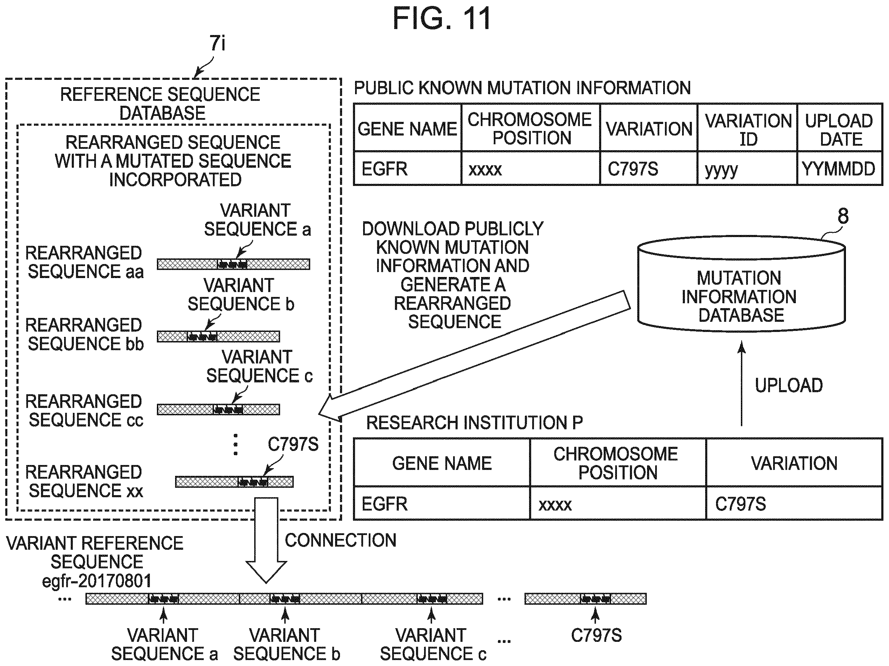

FIG. 11 is a conceptual diagram illustrating an outline of a method of generating a single mutation reference sequence and a conceptual diagram illustrating an example of a method of generating a mutation reference sequence using publicly known mutation information downloaded from an external mutation information database 8. In FIG. 11, a case in which information on a mutation "C797S" occurring in a gene "EGFR" at chromosome position "xxxx" is newly uploaded from a research institute P to an external mutation information database 8 and stored in the mutation information database 8 is used as an example. The information on the mutation "C797S" uploaded by the research institute P, which occurred at chromosome position "xxxx" of the gene with the gene name "EGFR", is registered as publicly known mutation information in the external mutation information database 8, associated with a mutation ID "yyyy" and an upload date "zz year z month z date", etc. The mutation illustrated here as newly uploaded information is a mutation in which the 797th amino acid residue of the protein "EGFR", which is the gene product transcribed and translated from the gene "EGFR", is replaced from cysteine to serine. Also, the external mutation information database 8 is not limited to such mutations, but information on polymorphisms, mutations, and methylation may be collected and stored.

The mutation information database 8 is an external public sequence information database, a publicly known mutation information database, etc. The public sequence information database includes the NCBI RefSeq (webpage,
www.ncbi.nlm.nih.gov/refseq/), NCBI GenBank (webpage,
www.ncbi.nlm.nih.gov/genbank/), UCSC Genome Browser, etc. Also, the publicly known mutation information database includes the COSMIC database (webpage, www.sanger.ac.uk/genetics/CGP/cosmic/), ClinVar database (webpage, www.ncbi.nlm.nih.gov/clinvar/), dbSNP (webpage, www.ncbi.nlm.nih.gov/SNP/), etc. In addition, the mutation information database 8 may be a publicly known mutation information database that includes frequency information for each race or animal species with respect to publicly known mutations. The publicly known mutation information database with such information includes HapMap Genome Browser release #28, Human Genetic Variation Browser (web page, www.genome.med.kyotou.ac.jp/SnpDB/index.html) and 1000 Genomes (web page, www.1000genomes.org/).

Referring again to FIG. 10, the process that the control unit 7f performs next is described. In step S62, the control unit 7f compares the acquired sequence with the reference sequence to identify the position on the reference sequence where a concordance rate between the acquired sequence and the reference sequence meets predetermined criteria. The above comparison is performed by mapping the acquired sequence to a plurality of positions on the reference sequence. The concordance rate is the ratio of the number of bases that match between the acquired sequence and the reference sequence to the number of bases contained in the acquired sequence. The identification of a position on the reference sequence is performed by calculating the concordance rate between the acquired sequence and the reference sequence at each of the mapped positions and identifying the position where the calculated concordance rate exceeds a predetermined threshold value.

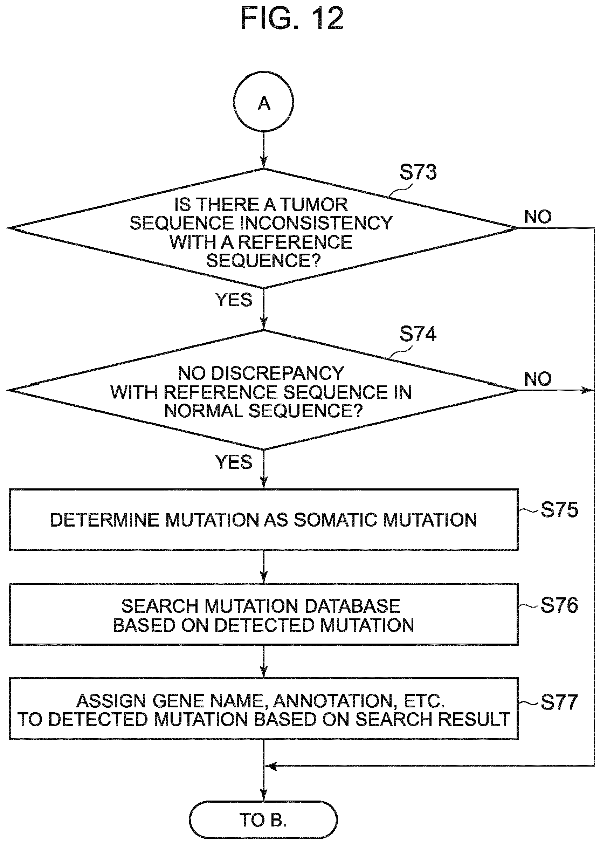

In step S63, the control unit 7f determines whether a plurality of positions on the reference sequence are identified, i.e., whether the concordance rate meets the predetermined criteria at a plurality of positions on the reference sequence. When the acquired sequence matches a single position on the reference sequence (in the case of "No"), the control unit 7f determines whether the positions on the reference sequence are identified for all the acquired sequences included in the 1 sequence data set extracted in step S42 in step S65. When the identification of positions is completed for all acquired sequences (in the case of "Yes"), the control unit 7f proceeds to step S73 (see FIG. 12). On the other hand, when the identification of positions is not completed for all acquired sequences (in the case of "No"), the control unit 7f returns the process to step S62 and continues the process.

In step S63, when multiple positions on the reference sequence are matched (in the case of "Yes"), the control unit 7f identifies the position with the highest concordance rate among the plurality of positions as the position on the reference sequence of the acquired sequence in step S64 and proceeds the process to step S65.

### Mutation Detection

### Detection of a Somatic Mutation

Next, with reference to FIG. 12, an example of a process by which the control unit 7f detects a somatic mutation is described. FIG. 12 is a flowchart illustrating a process by which the control unit 7f of the nucleic acid sequence analyzer 7 detects a somatic mutation.

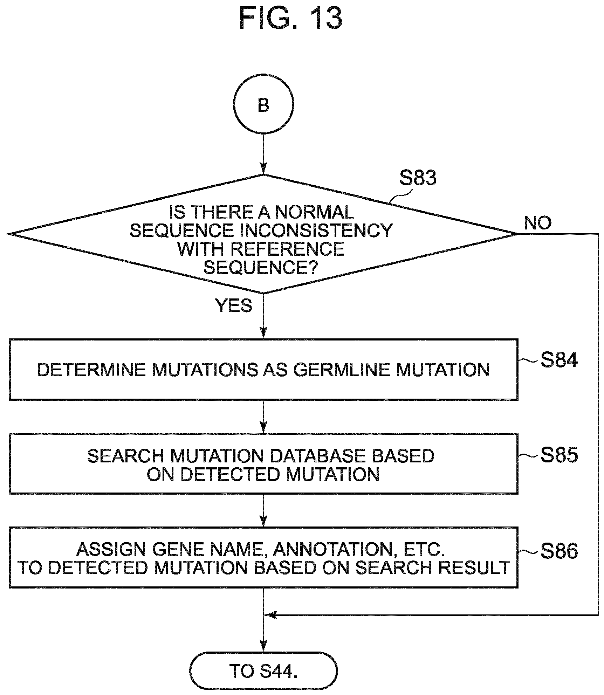

In step S73, the control unit 7f determines whether or not there is a discrepancy between a tumor sequence and the reference sequence at the position on the reference sequence identified in step S62 or S64 for the nucleic acid sequence data of the library sample derived from the tumor specimen (hereinafter referred to as "tumor sequence") among the plurality of nucleic acid sequence data included in the 1 sequence data set obtained in step S61 (see FIG. 10). When there is a mismatch (in the case of "Yes"), the control unit 7f proceeds the process to step S74, and when there is no mismatch (in the case of "No"), the process proceeds to step S83 (see FIG. 13). In step S74, the control unit 7f determines whether or not there is a discrepancy between a normal sequence and the reference sequence at the position on the reference sequence identified in step S62 or S64 for the nucleic acid sequence data of the library sample derived from the non-tumor specimen (hereinafter referred to as "normal sequence") among the nucleic acid sequence data included in the sequence data set referenced in step S73. When there is no discrepancy (in the case of "Yes"), the control unit 7f proceeds the process to step S75, and when there is a discrepancy (in the case of "No"), the process proceeds to step S83.

In step S75, the control unit 7f determines the mismatched base detected in step S73, i.e., a mutation, as a somatic mutation. In step S76, the control unit 7f searches the mutation information database stored in the mutation information database 8 based on the detected somatic mutation.

The mutation information stored in the mutation information database of the mutation information database 8 includes a mutation identifier (mutation ID), a gene name, mutation location information (e.g., "CHROM", and "POS"), "REF", "ALT", and "Annotation". The mutation ID is an identifier to identify a mutation. Among the mutation location information, "CHROM" indicates the chromosome number, "POS" indicates a position on the chromosome number. "REF" indicates the base in the wild type (Wild type), and "ALT" indicates the base after a mutation. "Annotation" indicates information about a mutation. "Annotation" may be information that indicates an amino acid mutation, such as "EGFR C2573G" and "EGFR L858R". For example, "EGFR C2573G" indicates that the cysteine at residue 2573 of the protein "EGFR" is replaced by glycine.

In step S77, based on the search result of step S76, the control unit 7f assigns mutation information such as a gene name, an annotation, etc. to the detected somatic mutation. In addition, in a first embodiment, steps S76 and S77 may be omitted. Detection of a Germline Mutation

Next, with reference to FIG. 13, an example of a process by which the control unit 7f detects a germline mutation is described. FIG. 13 is a flowchart illustrating a process by which the control unit 7f of the nucleic acid sequence analyzer 7 detects a germline mutation. In step S83, the control unit 7f determines whether or not there is a discrepancy between the normal sequence and the reference sequence at the position on the reference sequence identified in step S62 or S64 for the nucleic acid sequence data (normal sequence) of the library sample derived from the non-tumor specimen among the plurality of nucleic acid sequence data included in the 1 sequence data set obtained in step S61 (see FIG. 10). When there is a discrepancy (in the case of "Yes"), the control unit 7f proceeds the process to step S84, and when there is no discrepancy (in the case of "No"), the process proceeds to step S44 (see FIG. 7).

In step S84, the control unit 7f determines the mismatched base detected in step 83, i.e., a mutation, as a germline mutation. In step S85, the control unit 7f searches the mutation information database stored in the mutation information database 8 based on the detected germline mutation. In step S86, the control unit 7f assigns mutation information such as a gene name, an annotation, etc. to the detected mutation based on the search result of step S85. In addition, in a first embodiment, processes of steps S85 and S86 may be omitted.

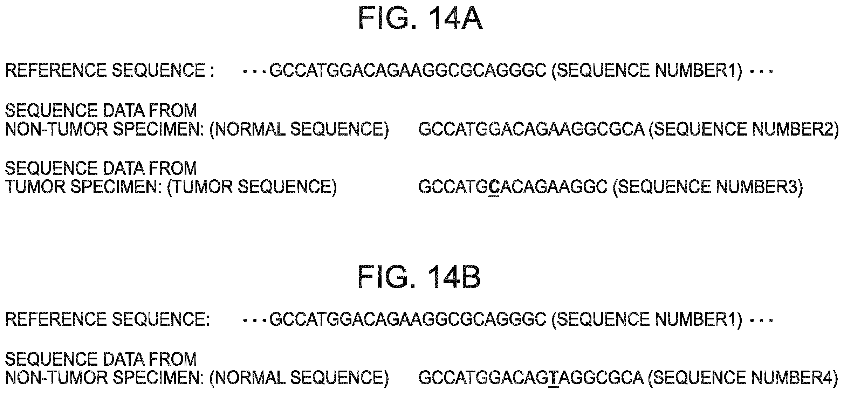

FIG. 14A is a diagram illustrating an example of a nucleic acid sequence with a somatic mutation, and FIG. 14B is a diagram illustrating an example of a nucleic acid sequence with a germline mutation. Referring to FIG. 14A, the sequence data derived from the non-tumor specimen (normal sequence) has no discrepancy with the reference sequence, but the sequence data derived from the tumor specimen (tumor sequence) has a base of discrepancy with the reference sequence (the reference sequence is G, whereas the tumor sequence is C), i.e., there is a mutation. Accordingly, the control unit 7f determines the mutation to be a somatic mutation in step S75 (see FIG. 12).

On the other hand, referring to FIG. 14B, the sequence data derived from the non-tumor specimen (normal sequence) contains a base of discrepancy with the reference sequence (the reference sequence is A, whereas the normal sequence is T), i.e., there is a mutation. Accordingly, the control unit 7f determines the mutation to be a germline mutation in step S84 (see FIG. 13).

### Analysis Result Report

Next, an example of an analysis report created in step S44 (see FIG. 7) is described. FIG. 15 is a diagram illustrating an example of an analysis report R1. As illustrated in FIG. 15, the analysis report R1 includes a summary report area S for posting a summary of an analysis result (hereinafter also referred to as "summary report area S") and a detailed report area D for posting details of the analysis result (hereinafter also referred to as "detailed report area D"). The summary report area S includes an area S1 illustrating attribute information in which information about the subject and the contents of the test is shown (hereinafter also referred to as "attribute information area S1"), an area S2 illustrating a list of all detected gene mutations (hereinafter also referred to as "gene mutation list area S2"). Also, the detailed report area D includes an area D1, in which a gene for which a somatic mutation is detected and detailed information on the mutation are shown (hereinafter also referred to as "gene mutation information area D1"), and an area D2, in which a gene for which a germline mutation is detected and detailed information on the mutation are shown (hereinafter also referred to as "germline mutation information area D2").

In the attribute information area S1, based on the information stored in the memory unit 7g in step S40, information to identify the patient, such as a patient identifier (patient ID), the name of the physician in charge, the name of the medical institution, etc., as well as information indicating a test item such as a gene panel, are displayed. In the gene mutation list area S2, regardless of a somatic mutation or a germline mutation, all detected gene mutations are indicated. In the example of the gene mutation list area S2, EGFR, BRAF, and BRCA1 represent gene names, and L585R, V600E, and K1183R indicate the mutation sites and substitution contents of the amino acid caused by mutations in each gene. In other words, EGFR_L585R indicates that the 585th codon of the EGFR gene is mutated from a nucleic acid sequence encoding leucine (L) to a nucleic acid sequence encoding arginine (R). In the various information displayed in the gene mutation list area S2, the information obtained by the control unit 7f in step S77 and step S86 is used.

### Effects of a First Embodiment

According to a first embodiment, in the case of a matched pair test in which nucleic acid sequence data of a tumor specimen and nucleic acid sequence data of a non-tumor specimen of 1 subject is analyzed as a set, the receiving device 6 receives a sequence data set including a plurality of nucleic acid sequence data obtained using the sequencer 2 corresponding to each of a plurality of library samples including the first library sample and the second library sample prepared from the specimen of the same subject and sequence run data including the link information indicating that the first library sample and the second library sample are prepared from the specimen of the same subject from the data transmitting device 5 via the network 11 and sends the sequence run data to the nucleic acid sequence analyzer 7 that analyzes the nucleic acid sequence. Therefore, even if the analysis request source facility 10 to operate the sequencer 2 is a different facility from the request destination facility 30 where the nucleic acid sequence analyzer 7 is installed, the nucleic acid sequence analyzer 7 can accurately and quickly extract the correct combination of respective nucleic acid sequence data corresponding to multiple library samples from the same subject from the sequence data set. Therefore, the correct combination of multiple nucleic acid sequence data may be analyzed at the request destination facility 30, and an analysis using multiple nucleic acid sequence data of the same subject may be performed accurately and quickly. In addition, in a first embodiment, the somatic mutation information obtained by analyzing the nucleic acid sequence data of the tumor specimen and the germline mutation information obtained by analyzing the nucleic acid sequence data of the non-tumor specimen are combined for comprehensive analysis, which enables an analysis to be performed based on more information and makes it easier to identify the appropriate treatment for the subject.

### Second Embodiment

In a first embodiment, a case in which the first library sample of DNA derived from a tumor cell collected from 1 subject and the second library sample of DNA derived from a non-tumor cell collected from the same subject are included in the sequence data set is described. In a second embodiment, a first library sample of DNA derived from a tumor cell collected from 1 subject and a second library sample of RNA derived from a tumor cell from the same subject are included in the sequence data set.

RNA may be produced due to a fusion gene mutation in DNA. Therefore, by identifying the nucleic acid sequence of RNA, it may be possible to identify the fusion gene mutation of DNA. In a second embodiment, information on a somatic mutation other than a fusion gene mutation may be obtained by an analysis result of the first sequence data corresponding to the first library sample, and information on a fusion gene mutation may be obtained by an analysis result of the second sequence data corresponding to the second library sample.

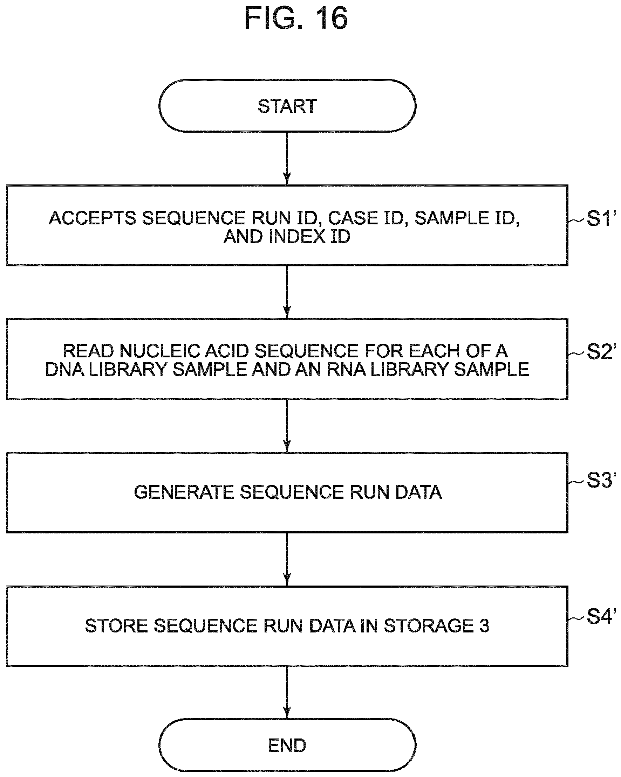

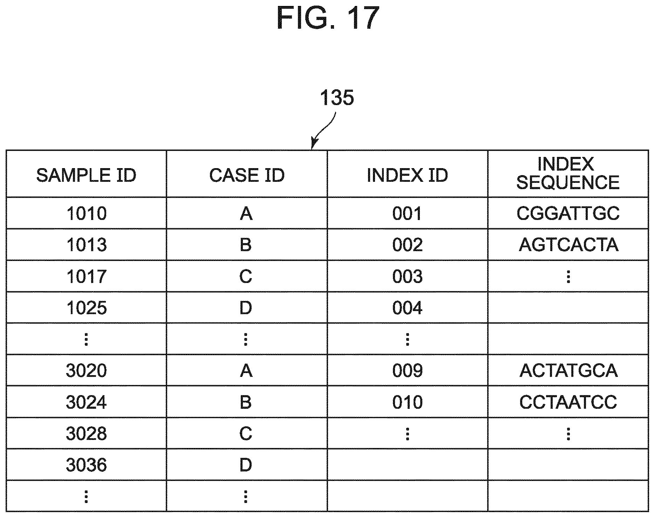

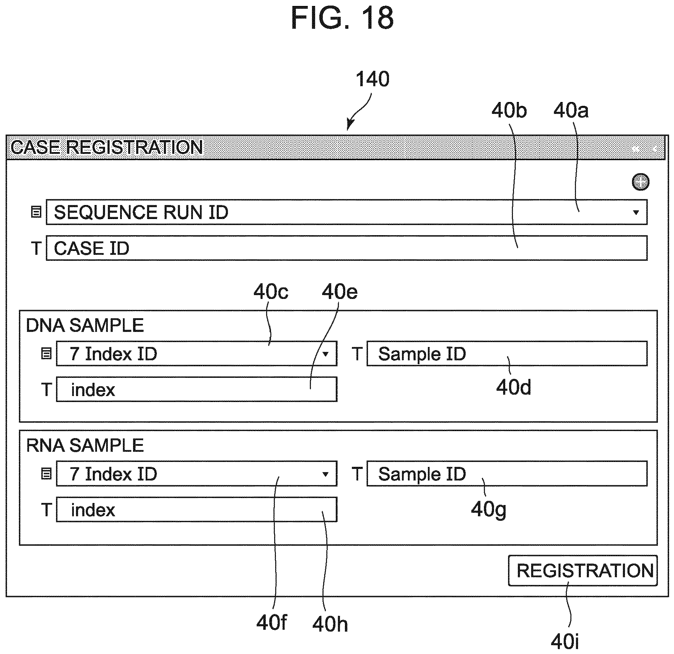

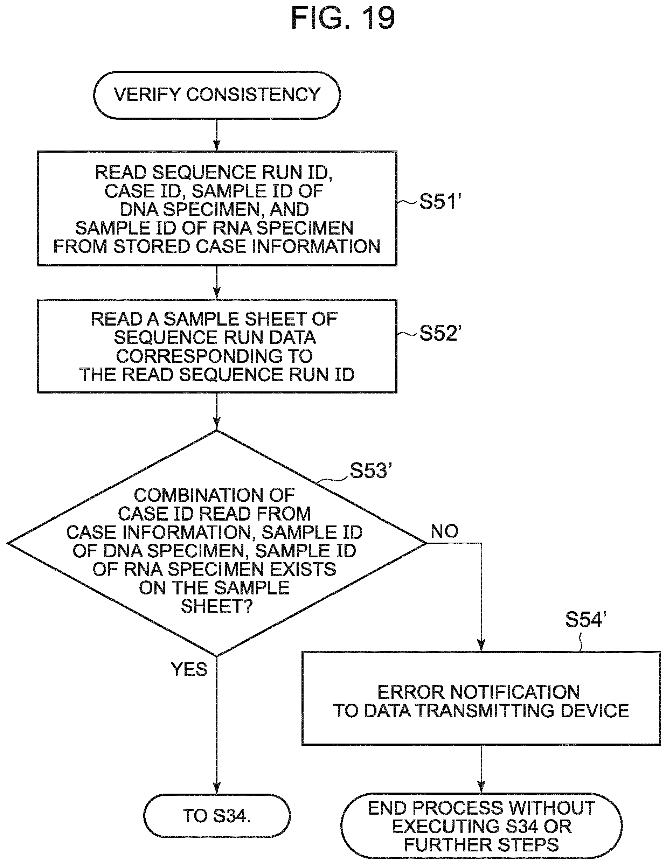

The schematic configuration of the nucleic acid information transmitting and receiving system 1 of a second embodiment is the same as that illustrated in FIG. 1. Also, the outlines of the processes executed by each control unit of the data transmitting device 5, the receiving device 6, and the nucleic acid sequence analyzer 7 are the same as those illustrated in FIG. 7. FIG. 16 is a flowchart illustrating a process executed by the sequencer 2 in a second embodiment, and FIG. 17 is a flowchart illustrating an example of a sample sheet 135 that may be employed in a second embodiment. FIG. 18 is a diagram illustrating an example of a case registration screen displayed on the display unit 5b of the data transmitting device 5 in a second embodiment. Also, FIG. 19 is a flowchart illustrating a detailed explanation of a process performed by the control unit 6f in step S33 (see FIG. 7) in a second embodiment.

Referring to FIG. 16, a process performed by the sequencer 2 is described. First, in step S1', the sequencer 2 accepts a sequence run ID, case ID, sample ID, and index ID and generates a sample sheet that is an electronic file. As in a first embodiment, the sequence run ID is information to identify sequence run data, and the sample sheet includes a case ID, sample ID, and index ID. One sample sheet is generated per sequence run, i.e., per cartridge. In one sequence run, i.e., one cartridge, nucleic acid sequences of multiple library samples (e.g., 16 samples) are read. The multiple library samples are prepared by pretreating each of the multiple samples (e.g., 16 samples) prepared from each of DNA of a tumor tissue and RNA of tumor tissue from multiple subjects (e.g., 8 subjects) with a reagent and by adding different index sequences to each of the multiple samples.

FIG. 17 is a diagram illustrating an example of a sample sheet 135. In the example illustrated in FIG. 17, a sample ID is associated with a case ID. Library samples with the same case ID are samples prepared from a specimen of the same subject, and the case ID on the sample sheet 135 is an example of the link information indicating that multiple library samples are prepared from the specimen of the same subject. Each sample ID is further associated with an index ID and an index sequence. The index sequence is information indicating the index sequence added to the library sample.

For example, a library sample with a sample ID 1010 is prepared from a specimen of a subject A with a specific disease and is a sample with an index ID 001. The index sequence of the library sample is CGGATTGC. A library sample with a sample ID 3020 is prepared from a specimen of the subject A with a specific disease and is a sample with an index ID 009. The index sequence of the prepared library sample is ACTATGCA. The library sample with the sample ID 1010 and the library sample with the sample ID 3020 have the same case ID (A), which indicates that both library samples are prepared from the specimen of the same subject A. Similarly, the library sample with the sample ID 1013 and the library sample with the sample ID 3024 have the same case ID (B), which indicates that both library samples are prepared from the specimen of the same subject B. Furthermore, in a second embodiment, when a sample ID is an ID starting from 1, it indicates that the corresponding library sample is derived from a tumor cell DNA, and when a sample ID is an ID starting from 3, it indicates that the corresponding library sample is derived from a tumor cell RNA. Therefore, by referring to the data on the sample sheet, multiple library samples derived from the same subject and information on whether each library sample is DNA-derived or RNA-derived may be identified.

Referring again to FIG. 16, the process that the sequencer 2 performs next is described. A user of the sequencer 2 dispenses a number of pre-prepared library samples (e.g., 16 samples) into each well of a single cartridge, sets the cartridge in the sequencer 2, and instructs to start sequence reading. When the start of sequence reading is instructed by the user, the sequencer 2 reads nucleic acid sequences for each of the plurality of library samples in step S2'. In a second embodiment or embodiments, the sequencer 2 reads a DNA library sample prepared from a tumor specimen and an RNA library sample prepared from the tumor specimen for each of the plurality of subjects. Then, the sequencer 2 generates sequence run data in step S3'. The sequence run data is the data in which the sample sheet 35 of the sequence run data 50 illustrated in FIG. 4 is replaced by the sample sheet 135. Then, in the next step S4', the generated sequence run data is stored in the storage 3, and the process is terminated.

In addition, the notation on the sample sheet may be any notation as long as it can recognize the information that can identify the corresponding subject and whether the library sample is derived from DNA or RNA for each library sample.

As illustrated in FIG. 18, for a case registration screen displayed by the control unit 5f of the data transmitting device 5 on the display unit 5b in a second embodiment, in comparison with the case registration screen 40 (see FIG. 8) in a first embodiment, a case registration screen 140, in which the phrase "normal specimen" is changed to "DNA specimen", and the phrase "tumor specimen" is changed to "RNA specimen", may be adopted.

As illustrated in FIG. 18, the case registration screen 140 displays a registration unit 40a for registering a sequence run ID, a registration unit 40b for registering a case ID, regarding a library sample derived from a DNA tumor specimen, a registration unit 40c for registering an index ID, a registration unit 40d for registering a sample ID, and a registration unit 40e for registering an index sequence, regarding a library sample derived from an RNA tumor specimen, a registration unit 40f for registering an index ID, a registration unit 40g for registering a sample ID, and a registration unit 40h for registering an index sequence, and a registration button 40i. The registration unit 40a, the registration unit 40c, and the registration unit 40f are configured with pull-down list formats, and when the pull-down list is expanded, a sequence run ID and an index ID, which are included in the sequence run data, to which the above-mentioned registered flag is not added, in the sequence run data that the control unit 5f reads from storage 3, are displayed in the list. The registration unit 40b, the registration unit 40d, the registration unit 40e, the registration unit 40g, and the registration unit 40h are configured so that the user inputs numerical values, letters, or symbols by using a keyboard. The user of the data transmitting device 5 operates the input unit 5a to input information into the registration units 40a to 40h for each subject, and when the registration of 1 subject is completed, the user selects the registration button 40i. The user of the data transmitting device 5 repeats an operation that enters information in the registration units 40a to 40h for each subject and selects the registration button 40i until inputs of all case IDs in 1 sequence run data are completed.

The registration units 40a to 40h may be configured in a pull-down list format, except for the registration unit 40a, the registration unit 40c, and the registration unit 40f, and the registration unit 40a, the registration unit 40c, and the registration unit 40f may be configured so that numerical values, etc., are entered. In addition, the registration unit 40e and the registration unit 40h may be configured so that when an index ID is input to the registration unit 40c or the registration unit 40f, the corresponding index sequence is read from the sequence run data and displayed in the registration unit 40e or the registration unit 40h. As a case registration screen, any screen that can register information that can identify the same subject corresponding to each of the library samples for the tumor specimen DNA and tumor specimen RNA may be employed.

FIG. 19 is a flowchart illustrating the details of a process performed by the control unit 6f in step S33 (see FIG. 7). In step S51', the control unit 6f obtains a sequence run ID, a case ID, a sample ID of a DNA specimen, and a sample ID of an RNA specimen from the stored case information. The sequence run ID is the information entered via the registration unit 40a of the case registration screen 140 (see FIG. 18), the case ID is the information entered via the registration unit 40b of the case registration screen 140, the sample ID of the DNA specimen is the information entered via the registration unit 40d, and the sample ID of the RNA specimen is the information entered via the registration unit 40g. In step S52', the control unit 6f reads the information on the sample sheet of the sequence run data that is assigned the same sequence run ID as the read sequence run ID. Then, in step S53', the control unit 6f determines whether or not the combination of the case ID, the sample ID of the DNA specimen, and the sample ID of the RNA specimen read from the case information in step S51' is present on the sample sheet.

When the control unit 6f makes a negative judgment (in the case of "No") in step S53', the control unit 6f performs an error notification indicating that the link information of the sequence run data and the case information are inconsistent to the data transmitting device 5 in step S54' and terminates the process without executing step S34' and thereafter (see FIG. 7).

The control unit 5f of the data transmitting device 5 that receives the error notification outputs the error information indicating that the link information of the sequence run data and the case information are inconsistent to the display unit 5b. The output of error information allows the user of the data transmitting device 5 to recognize that an error exists in at least one of the information on the sample sheet and the manually entered case information. On the other hand, when the control unit 6f of the receiving device 6 makes a positive judgment in step S53' (in the case of "Yes"), the process returns to step S34 (see FIG. 7).

According to a second embodiment or embodiments, in step S53', it is determined whether or not the link information of the sequence run data and the case information are consistent, and when the two pieces of information are not consistent, the process is terminated without moving on to the next step. Therefore, in each subject, the nucleic acid sequence data derived from the DNA specimen and the nucleic acid sequence data derived from the RNA specimen may be precisely linked to the subject. Thus, even in the case of a matched pair test, in which multiple nucleic acid sequence data derived from the same subject are analyzed, an analysis mistake by mistaking the nucleic acid sequence data may be reliably prevented.

### Effects of a Second Embodiment

According to a second embodiment, when performing a matched pair test in which nucleic acid sequence data of DNA derived from a tumor specimen and nucleic acid sequence data of RNA derived from a tumor specimen of 1 subject are analyzed as a set, the receiving device 6 receives the sequence data including the sequence data set including a plurality of nucleic acid sequence data obtained using the sequencer 2 corresponding to each of a plurality of library samples including the first library sample and the second library sample prepared from the specimen of the same subject and the link information indicating that the first library sample and the second library sample are prepared from the specimen of the same subject from the data transmitting device 5 via the network 11 and sends the sequence run data to the nucleic acid sequence analyzer 7 that analyzes the nucleic acid sequence. Therefore, even if the analysis request source facility 10 to operate the sequencer 2 is a different facility from the request destination facility 30 where the nucleic acid sequence analyzer 7 is installed, the nucleic acid sequence analyzer 7 can accurately and quickly extract the correct combination of respective nucleic acid sequence data corresponding to multiple library samples from the same subject from the sequence data set. Therefore, the correct combination of multiple nucleic acid sequence data may be analyzed at the request destination facility 30, which makes it possible to perform an analysis using multiple nucleic acid sequence data of the same subject accurately and quickly. In addition, in a second embodiment, information on a somatic mutation other than a fusion gene mutation obtained by analyzing the nucleic acid sequence data of DNA of the tumor specimen and information on a fusion gene mutation obtained by analyzing nucleic acid sequence data of RNA of the tumor specimen are combined for a comprehensive analysis, allowing analysis based on more information and making it easier to identify a suitable treatment for the subject.

### Third Embodiment

In first and second embodiments, the case in which two library samples derived from the same subject exist in the sequence data set is described. In a third embodiment, there are three library samples derived from the same subject in a sequence data set. The three library samples are a library sample derived from a DNA of a tumor specimen, a library sample derived from an RNA of a tumor specimen, and a library sample derived from a DNA of a non-tumor specimen, respectively.

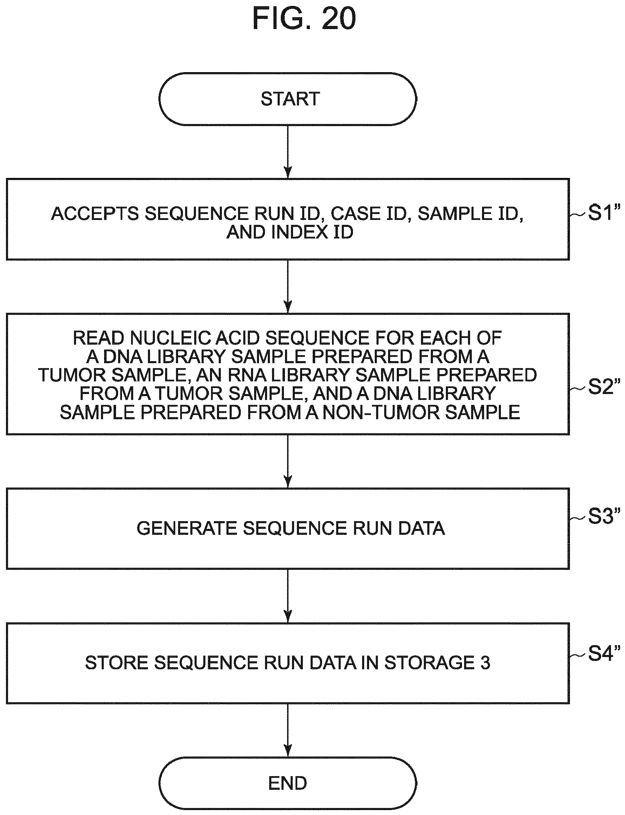

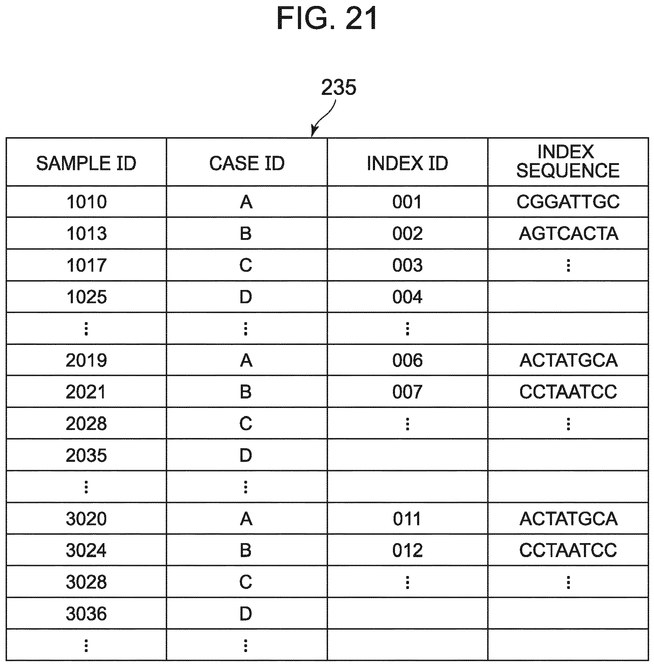

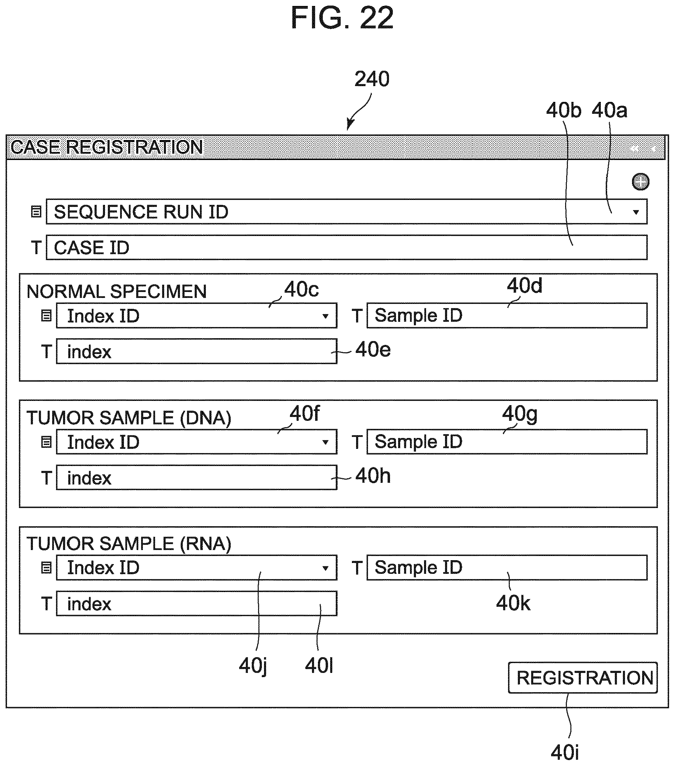

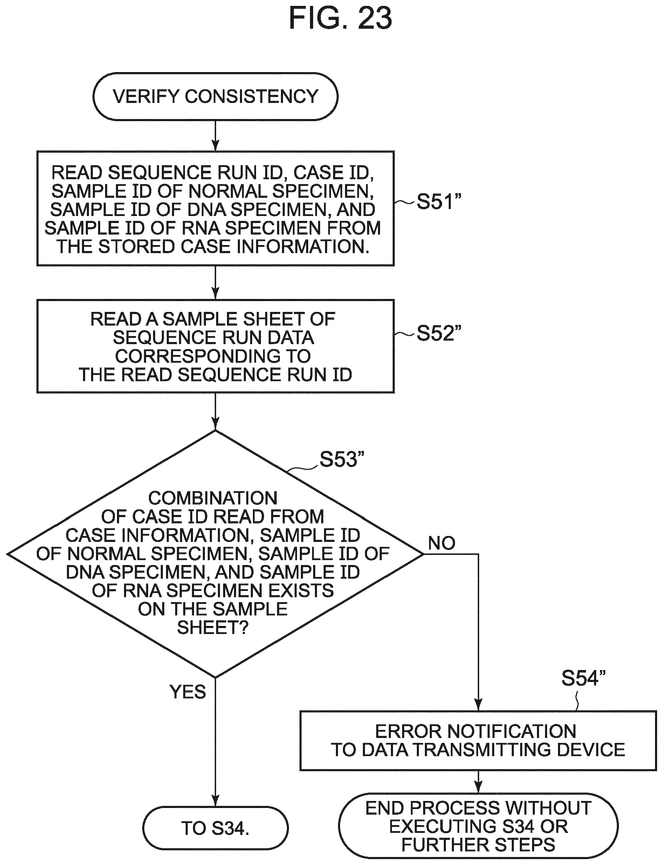

A schematic diagram of the nucleic acid information transmitting and receiving system 1 of a third embodiment is the same as that illustrated in FIG. 1. Also, the outlines of processes executed by each control unit of the data transmitting device 5, the receiving device 6, and the nucleic acid sequence analyzer 7 are the same as those illustrated in FIG. 7. FIG. 20 is a flowchart illustrating a process executed by the sequencer 2 in a third embodiment, and FIG. 21 is a diagram illustrating an example of a sample sheet 235 that may be employed in a third embodiment. Also, FIG. 22 is a diagram illustrating an example of a case registration screen displayed on the display unit 5b of the data transmitting device 5 in a third embodiment. Furthermore, FIG. 23 is a flowchart illustrating a process in detail performed by the control unit 6f in step S33 (see FIG. 7) in a third embodiment.

Referring to FIG. 20, a process performed by the sequencer 2 is described. First, in step S1", the sequencer 2 accepts a sequence run ID, case ID, sample ID, and index ID and generates a sample sheet that is an electronic file. As in Embodiments 1 and 2, the sequence run ID is information that identifies sequence run data, and the sample sheet contains the case ID, sample ID, and index ID. One sample sheet is generated per sequence run, i.e., per cartridge. In one sequence run, i.e., one cartridge, nucleic acid sequences of multiple library samples (e.g., 15 samples) are read. The multiple library samples are prepared by pretreating each of the multiple samples (e.g., 15 samples) prepared from each of DNA of tumor tissue, RNA of tumor tissue, and DNA of non-tumor tissue from multiple subjects (e.g., 5 subjects) with a reagent and adding different index sequences to multiple samples.

FIG. 21 is a diagram illustrating an example of a sample sheet 235. In the example illustrated in FIG. 21, a sample ID is associated with a case ID. Library samples with the same case ID are samples prepared from a specimen of the same subject, and the case ID on the sample sheet 235 is an example of the link information indicating that multiple library samples are prepared from the specimen of the same subject. Each sample ID is further associated with an index ID and an index sequence. The index sequence is information indicating the index sequence added to the library sample.

For example, a library sample with a sample ID 1010 is prepared from a specimen of a subject A with a specific disease and is a sample with an index ID 001. The index sequence of the prepared library sample is CGGATTGC. A library sample with a sample ID 2019 is prepared from the specimen of the subject A with a specific disease and is a sample with an index ID 006. The index sequence of the prepared library sample is ACTATGCA. A library sample with a sample ID 3020 is prepared from the specimen of the subject A with a specific disease and is a sample with an index ID 011. The library sample with the sample ID 1010, the library sample with the sample ID 2019, and the library sample with the sample ID 3020 have the same case ID (A), indicating that each library sample is prepared from the specimen of the same subject A. Similarly, a library sample with a sample ID 1013, a library sample with a sample ID 2021, and a library sample with a sample ID 3024 have the same case ID (B), indicating that each library sample is prepared from the specimen of the same subject B. Furthermore, in a third embodiment, when a sample ID is an ID starting from 1, it indicates that the corresponding library sample is derived from a DNA of a tumor cell, when a sample ID is an ID starting from 2, it indicates that the corresponding library sample is derived from a DNA of a non-tumor cell, and when a sample ID is an ID starting from 3, it indicates that the corresponding library sample is derived from an RNA of a tumor cell. Thus, by referring to the data on the sample sheet, it is possible to identify multiple library samples derived from the same subject and whether each library sample is DNA-derived, RNA-derived, or non-tumor-derived.

Referring again to FIG. 20, next, the process that the sequencer 2 performs next is described. A user of the sequencer 2 dispenses a number of pre-prepared library samples (e.g., 15 samples) into each well of a single cartridge, sets the cartridge in the sequencer 2, and instructs to start sequence reading. When the start of sequence reading is instructed by the user, the sequencer 2 reads the nucleic acid sequences for each of the plurality of library samples in step S2". In a third embodiment, the sequencer 2 reads, for each of the plurality of subjects, a DNA library sample prepared from a tumor specimen, an RNA library sample prepared from a tumor specimen, and a DNA library sample prepared from a non-tumor specimen. Then, the sequencer 2 generates sequence run data in step S3". The sequence run data is the data in which the sample sheet 35 of the sequence run data 50 illustrated in FIG. 4 is replaced by a sample sheet 235. Then, in the next step S4", the generated sequence run data is stored in the storage 3, and the process is terminated. In addition, the notation on the sample sheet may be any notation that recognizes, for each library sample, information that identifies the corresponding subject and whether the library sample is DNA-derived, RNA-derived, or non-tumor-derived.

As illustrated in FIG. 22, in a third embodiment, for a case registration screen displayed by the control unit 5f of the data transmitting device 5 on the display unit 5b, in comparison with the case registration screen 40 of a first embodiment (see FIG. 8), the phrase "tumor specimen" is changed to "tumor specimen (DNA)", and a case registration screen 240 with the addition of the registration units 40j to 401 to register information on a library sample derived from an RNA of a tumor specimen may be adopted.

As illustrated in FIG. 22, the case registration screen 240 displays a registration unit 40a for registering a sequential run ID, a registration unit 40b for registering a case ID, for a library sample derived from a DNA of a non-tumor specimen, a registration unit 40c for registering an index ID, a registration unit 40d for registering a sample ID, and a registration unit 40e for registering an index sequence, for a library sample derived from a DNA of a tumor specimen, a registration unit 40f for registering an index ID, a registration unit 40g for registering a sample ID, and a registration unit 40h for registering an index sequence, for a library sample derived from an RNA of a tumor specimen, a registration unit 40j for registering an index ID, a registration unit 40k for registering a sample ID, and a registration unit 40l for registering an index sequence, and a registration button 40i. The registration unit 40a, the registration unit 40c, the registration unit 40f, and the registration unit 40j are configured in pull-down list formats, and when the pull-down list is expanded, a sequence run ID and an index ID included in the sequence run data, to which the aforementioned registered flag is not added in the sequence run data read from the storage 3 by the control unit 5f, are displayed in the list. The registration unit 40b, the registration unit 40d, the registration unit 40e, the registration unit 40g, the registration unit 40h, the registration unit 40k, and the registration unit 401 are configured so that the user operates a keyboard to input numerical values, letters, or symbols. The user of the data transmitting device 5 operates the input unit 5a to input information into the registration units 40a to 40l for each subject, and when the registration of 1 subject is completed, the user selects the registration button 40i. The user of the data transmitting device 5 repeats an operation to input information into the registration units 40a to 40l for each subject and selects the registration button 40i until inputs of all case IDs included in 1 sequence run data are completed.

The registration units 40a to 401 may be configured in pull-down list formats other than the registration unit 40a, the registration unit 40c, the registration unit 40f, and the registration unit 40j, and the registration unit 40a, the registration unit 40c, the registration unit 40f, and the registration unit 40j may be configured so that numerical values, etc. are input. In addition, the registration unit 40e, the registration unit 40h, and the registration unit 40l may be configured so that when an index ID is input to the registration unit 40c, the registration unit 40f, or the registration unit j, the corresponding index sequence is read from the sequence run data and displayed on the registration unit 40e, the registration unit 40f, or the registration unit 40l. As for the case registration screen, any screen that can register information that can identify the same corresponding subject for each library sample of DNA of a tumor specimen, RNA from a tumor specimen, and DNA from a non-tumor specimen may be employed.

FIG. 23 is a flowchart illustrating the details of a process performed by the control unit 6f in step S33 (see FIG. 7). In step S51 ", the control unit 6f reads a sequence run ID, case ID, sample ID of a DNA specimen, sample ID of an RNA specimen, and sample ID of a non-tumor specimen from the stored case information. The sequence run ID is the information entered via the registration unit 40a of the case registration screen 240 (see FIG. 22), the case ID is the information entered via the registration unit 40b, the sample ID of the non-tumor specimen is the information entered via the registration unit 40d, the sample ID of the DNA specimen is the information entered via the registration unit 40g, and the sample ID of the RNA specimen is the information entered via the registration unit 40k. In step S52", the control unit 6f reads the information on the sample sheet of the sequence run data to which the same sequence run ID as the read sequence run ID is assigned. Then, in step S53", the control unit 6f determines whether or not the combination of the case ID, sample ID of the DNA specimen, sample ID of the RNA specimen, and sample ID of the non-tumor specimen read from the case information in step S51" is present on the sample sheet.

When the control unit 6f makes a negative judgment (in the case of "No") in step S53", the control unit 6f performs an error notification indicating that the link information of the sequence run data and the case information are inconsistent to the data transmitting device 5 in step S54" and terminates the process without executing step S34 and thereafter (see FIG. 7). The control unit 5f of the data transmitting device 5 that receives the error notification outputs the error information indicating that the link information of the sequence run data and the case information are inconsistent to the display unit 5b. The output of error information allows the user of the data transmitting device 5 to recognize that an error exists in at least one of the information on the sample sheet and the manually entered case information. On the other hand, when the control unit 6f of the receiving device 6 makes a positive judgment in step S53" (in the case of "Yes"), the process returns to step S34 (see FIG. 7).

According to a third embodiment or embodiments, in step S53", it determines whether or not the link information of the sequence run data and the case information are consistent, and when the two information are inconsistent, the process is terminated without moving on to the next step. Therefore, in each subject, the nucleic acid sequence data derived from the DNA specimen of the tumor specimen, the nucleic acid sequence data derived from the RNA specimen of the tumor specimen, and the nucleic acid sequence data derived from the non-tumor specimen may be accurately associated with the subject. Therefore, even in the case of a matched pair test in which multiple nucleic acid sequence data derived from the same subject are analyzed, incorrect analysis by mistaking the nucleic acid sequence data may be reliably prevented.

### Effects of a Third Embodiment

According to a third embodiment, when performing a matched pair test in which nucleic acid sequence data of DNA derived from a tumor specimen, nucleic acid sequence data of RNA derived from a tumor specimen, and nucleic acid sequence data of DNA derived from a non-tumor specimen of 1 subject are analyzed as a set, the receiving device 6 receives the sequence data set containing a plurality of nucleic acid sequence data obtained using the sequencer 2 corresponding to respective multiple library samples including a first library sample, a second library sample, and a third library sample prepared from the specimen of the same subject and the sequence run data including the link information indicating that the first library sample, the second library sample, and the third library sample are prepared from the specimen of the same subject from the data transmitting device 5 via network 11 and sends the sequence run data to the nucleic acid sequence analyzer 7, which analyzes the nucleic acid sequence. Therefore, even if the analysis request source facility 10 to operate the sequencer 2 is a different facility from the request destination facility 30 where the nucleic acid sequence analyzer 7 is installed, the nucleic acid sequence analyzer 7 can accurately and quickly extract the correct combination of respective nucleic acid sequence data corresponding to multiple library samples from the same subject from the sequence data set. Therefore, the multiple nucleic acid sequence data with the correct combination may be analyzed at the request destination facility 30, which makes it possible to perform an analysis using multiple nucleic acid sequence data of the same subject accurately and quickly. In addition, in a third embodiment, information on a somatic mutation other than a fusion gene mutation obtained by analyzing nucleic acid sequence data of a DNA of a tumor specimen, information on a fusion gene mutation obtained by analyzing nucleic acid sequence data of an RNA of a tumor specimen, and information on a germline mutation obtained by analyzing nucleic acid sequence data of a DNA of a non-tumor specimen are combined in a comprehensive analysis, allowing analysis based on more information and making it easier to identify a suitable treatment for a subject.

### Fourth Embodiment

In a first embodiment, the case in which information is exchanged between the data transmitting device 5 and the nucleic acid sequence analyzer 7 via the receiving device 6 is described, but the receiving device 6 and the nucleic acid sequence analyzer 7 may be configured with a single computer.

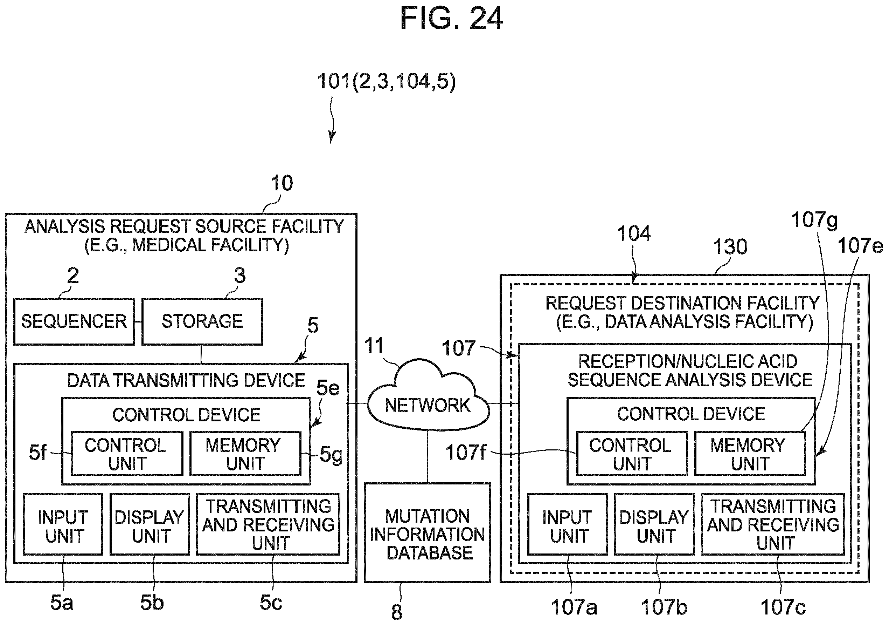

FIG. 24 is a schematic diagram of a nucleic acid information transmitting and receiving system 101 installed at each facility according to a fourth embodiment. The nucleic acid information transmitting and receiving system 101 consists of a sequencer 2, a storage (storage device) 3, a data transmitting device 5, and a reception/analysis system 104, and the reception/analysis system 104 has a reception/analysis device 107. The data transmitting device 5 and the reception/analysis device 107 are connected to each other via a network 11, which is the internet. The network 11 is further connected to a mutation information database 8. The hardware configurations of the sequencer 2, the storage (storage device) 3, and the data transmitting device 5 are the same as in a first embodiment. The data transmitting device 5 sends and receives data to and from the reception/analysis device 107 via the network 11.

The reception/analysis device 107 is installed at a request destination facility 130, e.g., a data analysis facility. The analysis request source facility 10 and the request destination facility 130 may be different facilities. The reception/analysis device 107 may be a computer that constitutes a cloud system. The data analysis facility may be a facility of a cloud service provider or a facility of a company that provides nucleic acid sequence analysis services. The reception/analysis device 107 is a computer. The reception/analysis device 107 includes an input unit 107a, a display unit 107b, a transmitting/receiving unit 107c, and a control device 107e. The control device 107e includes a control unit 107f and a memory unit 107g. The hardware configurations of the input unit 107a, the display unit 107b, the transmitting/receiving unit 107c, and the control device 107e are the same as those of the input unit 5a, the display unit 5b, the transmitting/receiving unit 5c, and the control device 5e of the data transmitting device 5, respectively. The reception/analysis device 107 is able to access the mutation information database 8 via the network 11.

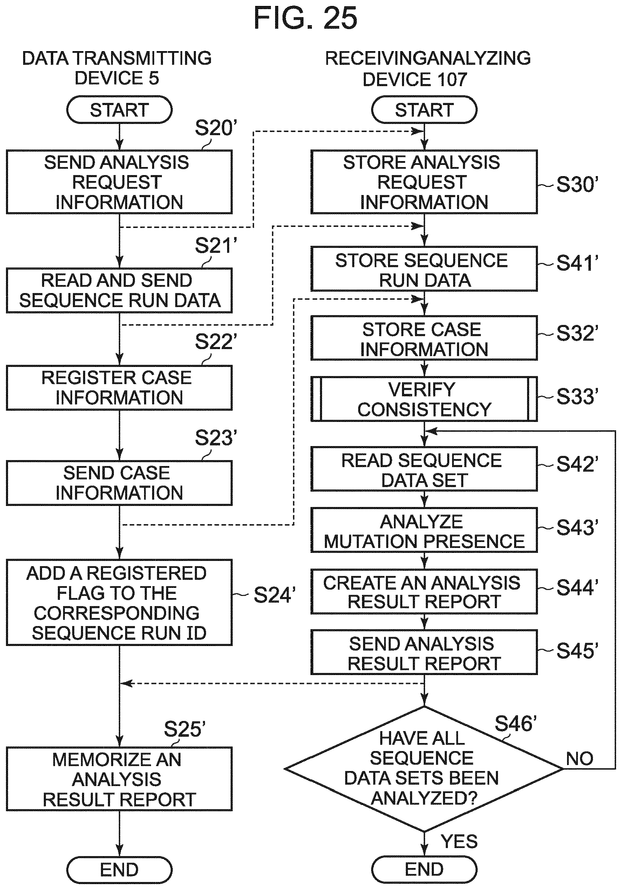

FIG. 25 is a flowchart illustrating processes executed by each control unit of the data transmitting device 5 and the reception/analysis device 107. Referring to FIG. 25, the process executed by the control unit 5f of the data transmitting device 5 is explained first. When the control unit 5f receives an analysis instruction for the sequence run data stored in the storage 3 from a user of the data transmitting device 5, the control unit 5f sends the analysis request information to the receiving device 6 in step S20'. In step S21', the control unit 5f reads the sequence run data from the storage 3 and sends it to the reception/analysis device 107.

In step S22', the control unit 5f makes the display unit 5b display a case registration screen and accepts registration of case information. For the case registration screen, the case registration screens 40, 140, or 240 illustrated in Embodiments 1 to 3 may be employed.

When the process of step S22' is completed, the control unit 5f sends the case information entered in the case registration screen in step S22' to the reception/analysis device 107 in step 23'. In step S24', the control unit 5f adds a flag indicating the registration is completed to the sequence run ID corresponding to the sequence run data sent to the reception/analysis device 107 in step S21'.

Next, a process performed by the control unit 107f of the reception/analysis device 107 is described. When there is a transmission of an analysis request information from the data transmitting device 5, the control unit 107f receives the analysis request information and stores it in the memory unit 107g in step S30'. When there is a transmission of sequence run data from the data transmitting device 5, the control section 107f receives the sequence run data and stores it in the memory unit 107g in step S41'. Also, when there is a transmission of the case information from the data transmitting device 5, the control unit 107f receives the case information and stores it in the memory unit 107g in step S32'. In the subsequent step S33', the control unit 107f conducts verification of consistency and determines whether or not the link information contained in the sequence run data stored in step S41' is consistent with the information contained in the case information stored in step S32'.

In step S42', the control unit 107f reads from 1 sequence data set from the stored sequence run data. As described above, since the sequence data set contains multiple nucleic acid sequence data corresponding to the same case ID, the control unit 107f can extract the multiple nucleic acid sequence data corresponding to the same case ID as 1 sequence data set using the case ID, which is the link information, as a search key.

In step S43', the control unit 107f analyzes the presence and absence of a mutation for each nucleic acid sequence data in the sequence data set extracted in step S42' using the information on the nucleic acid sequences of the tumor cells in the mutation information database 8. In step 44', the control unit 107f creates an analysis result report based on the presence or absence of the mutation. In step S45', the control unit 107f sends the analysis result report to the data transmitting device 5. In step S46', the control unit 107f determines whether or not all sequence data sets included in the sequence run data stored in step S41' have been analyzed. When all sequence data sets have been analyzed (in the case of "Yes"), the control unit 107f terminates the process, and when all sequence data sets have not been analyzed (in the case of "No"), the control unit 107f returns the process to step S42' and performs the processes of steps S42' to S46' again.

On the other hand, the control unit 5f of the data transmitting 5 receives the analysis result report and stores it in the storage unit 5g in step S25', and the process ends, allowing the physician in charge of the subject to display and view the analysis report stored in the storage unit 5g on the display unit 5b at any time.

### Effects of a Fourth Embodiment

According to a fourth embodiment, the hardware configuration of the reception/analysis system 104 is simplified. In addition, since reception and analysis of the sequence run data may be done with the same computer, the time required for sending and receiving sequence run data may be reduced, and the communication speed reduction due to the large volume of data flowing over the network 11 may be suppressed.

### Fifth Embodiment

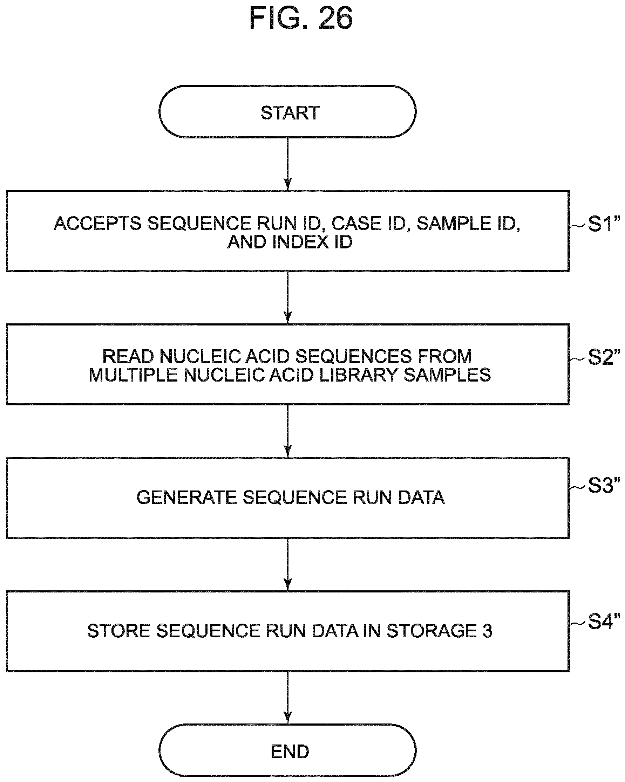

A fifth embodiment is an embodiment that encompasses embodiments 1 to 4 and their variations. For the schematic configuration of the nucleic acid information transmitting and receiving system 101, either the configuration of Embodiments 1 to 3 (see FIG. 1) or the configuration of a fourth embodiment (see FIG. 24) may be adopted. FIG. 26 is a flowchart illustrating a process performed by the sequencer 2 in a fifth embodiment. Referring to FIG. 26, the process executed by the sequencer 2 is described. First, in step S1", the sequencer 2 accepts a sequence run ID, a case ID, a sample ID, and an index ID, and generates a sample sheet, which is an electronic file.

Next, a user of the sequencer 2 dispenses multiple pre-prepared library samples into each well of one cartridge, sets the cartridge in the sequencer 2, and instructs to start sequence reading. When the start of sequence reading is instructed by the user, the sequencer 2 reads the nucleic acid sequences for each of the multiple library samples in step S2". In a fifth embodiment, the sequencer 2 reads the nucleic acid sequences of the multiple library samples collected and prepared from the same subject for each of the multiple subjects. Then, in step S3", the sequencer 2 generates sequence run data. Then, in the next step S4", the sequencer 2 stores the generated sequence run data in the storage 3 and terminates the process.

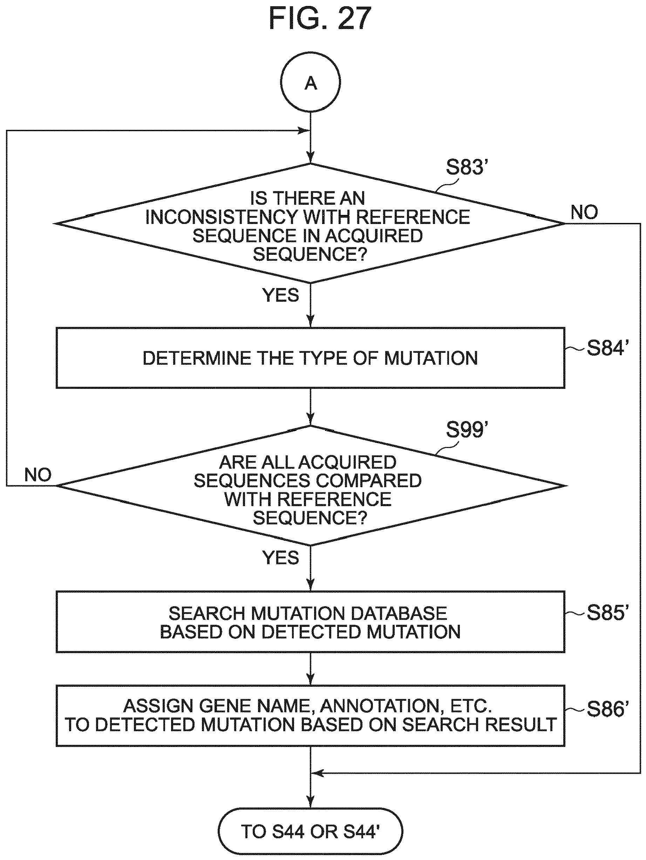

FIG. 27 is a flowchart illustrating a process of determining the type of a mutation in the multiple nucleic acid sequence data included in 1 sequence data set extracted in step S42 (see FIG. 7) or step S42' (see FIG. 25). Referring to FIG. 27, the process performed by the control unit 7f or the control unit 107f is described. In step S83', the control unit 7f or the control unit 107f determines whether or not there is a discrepancy between the acquired sequence and the reference sequence for 1 acquired sequence in the multiple nucleic acid sequence data included in the 1 sequence data set acquired in step S61 (see FIG. 10). When there is a discrepancy (in the case of "Yes"), the control unit 7f or the control unit 107f proceeds the process to step S84', and when there is no discrepancy (in the case of "No"), the control unit 7f or the control unit 107f proceeds the process to step S44 (see FIG. 7) or step S44' (see FIG. 25).

In step S84', the control unit 7f or the control unit 107f determines the mismatched base detected in step S83', i.e., the type of a mutation. In step S99', the control unit 7f or the control unit 107f determines whether or not all of the multiple nucleic acid sequence data included in the acquired 1 sequence data set have been compared with the reference sequence. When it is determined that all of the nucleic acid sequence data have been compared (in the case of "Yes"), the control unit 7f or the control unit 107f advances the process to step S85'. When it is determined that all of the nucleic acid sequence data have not been compared (in the case of "No"), the control unit 7f or the control unit 107f returns the process to step S83'.

In step S85', the control unit 7f or the control unit 107f searches the mutation information database stored in the mutation information database 8 based on each detected mutation. In step S86', the control unit 7f assigns a gene name, annotation, etc. to each detected mutation based on the search result of step S85'. In a fifth embodiment, the processes of steps S85' and S86' may be omitted.

### Effects of a Fifth Embodiment

According to a fifth embodiment, even if the analysis request resource facility 10 to operate the sequencer 2 is a different facility from the request destination facility 30 where the nucleic acid sequence analyzer 7 is installed or the request destination facility 130 where the reception/analysis device 107 is installed, the nucleic acid sequence analyzer 7 or the reception/analysis device 107 can accurately and quickly extract the correct combination of respective nucleic acid sequence data corresponding to multiple library samples of the same subject from the sequence data set. Therefore, multiple nucleic acid sequence data of the correct combination may be analyzed at the request destination facilities 30 or 130, enabling accurate and rapid analysis using multiple nucleic acid sequence data from the same subject.

### Sixth Embodiment

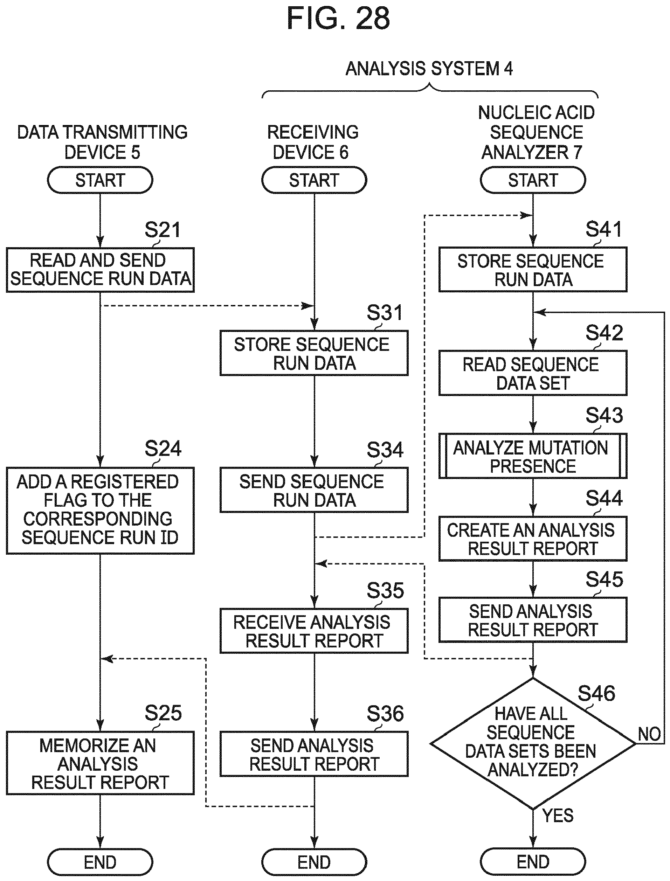

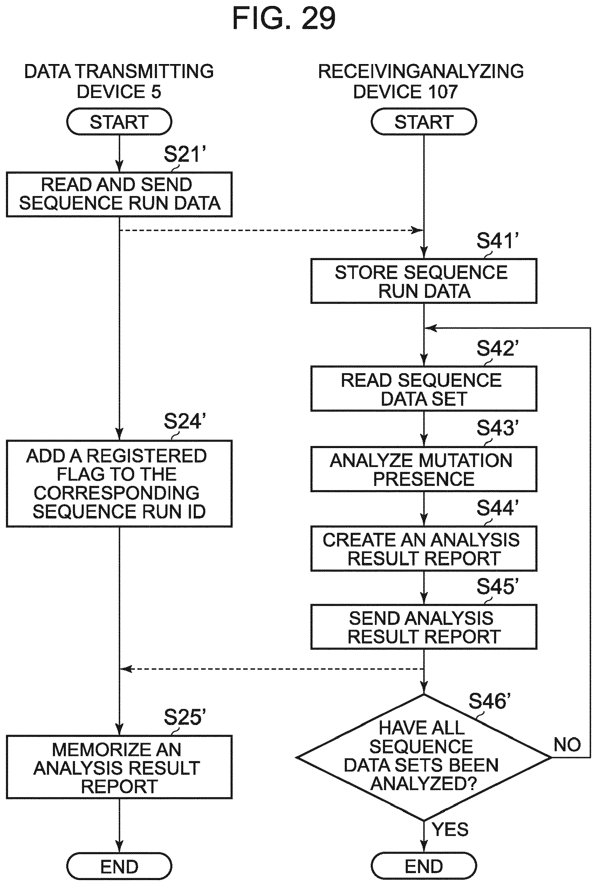

In a fifth embodiment, the processes of sending analysis request information and consistency verification are executed, but a sixth embodiment differs from a fifth embodiment in that the processes of sending analysis request information and consistency verification are not executed. FIG. 28 is a flowchart illustrating processes performed by each control unit of the data transmitting device 5, the receiving device 6, and the nucleic acid sequence analyzer 7 in a sixth embodiment. FIG. 29 is a flowchart illustrating processes executed by each of the control units of the data transmitting device 5 and the reception/analysis device 107 in a sixth embodiment. As illustrated in FIG. 28, in a sixth embodiment, the control unit 5f of the data transmitting device 5 performs processes of steps S21, S24, and S25 but does not perform processes of steps S20, S22, and S23 (see FIG. 7). The control unit 6f of the receiving device 6 performs processes of steps S31, S34, S35, and S36 but does not perform processes of steps S30, S32, and S33 (see FIG. 7). The control unit 7f of the nucleic acid sequence analyzer 7 performs processes of steps S41, S42, S43, S44, S45, and S46 but does not perform the process of step S40 (see FIG. 7). As illustrated in FIG. 29, in a sixth embodiment, the control unit 5f of the data transmitting device 5 performs processes of steps S21', S24', and S25' but does not perform processes of steps S20', S22', and S23' (see FIG. 25). The control unit 107f of the reception/analysis device 107 performs processes of steps S41', S42', S43', S44', S45', and S46' but does not perform processes of steps S30', S32', and S33' (see FIG. 25).

The invention is not limited to the above embodiments and variations thereof, and various improvements and changes are possible within the scope of the claims of the present application and their equivalents.

For example, the analysis system 4 may be configured with three or more computers. Also, the first library sample may be prepared from a specimen collected from one tumor tissue of one subject, and the second library sample may be prepared from a specimen collected from a tumor tissue different from the one tumor tissue of the same subject. For example, the first library sample may be prepared from a specimen collected from the colon of one subject, and the second library sample may be prepared from a specimen collected from the stomach of the same subject.

## Claims

1. A control method of controlling a computer to analyze, at a second facility, nucleic acid sequence data obtained, at a first facility, by a sequencer that reads a nucleic acid sequence, for a gene panel test, comprising
receiving, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject;
analyzing a first sequence data and a second sequence data corresponding to each of the first library sample and the second library sample linked by the link information; and
outputting analysis information based on an analysis result of the first sequence data and an analysis result of the second sequence data.

2. The control method according to claim 1, wherein
the receiving comprises receiving, by a first computer, the sequence data set and the link information, and the control method further comprising
sending, by the first computer, the received sequence data set and the link information to a second computer, wherein
the analyzing comprises, by the second computer, analyzing the first sequence data and the second sequence data, and
the outputting comprises, by the second computer, outputting the analysis information.

3. The control method according to claim 1, wherein
the receiving the sequence data set and the link information, the analyzing the first sequence data and the second sequence data, and the outputting the analysis information are executed by a computer.

4. The control method according to any one of claims 1 to 3, wherein
the first library sample is prepared from a tumor specimen of the subject, and the second library sample is prepared from a non-tumor specimen of the subject, and
the analysis information comprises somatic mutation information based on an analysis result of the first sequence data and germline mutation information based on an analysis result of the second sequence data.

5. The control method according to any one of claims 1 to 3, wherein
the first library sample is prepared from deoxyribonucleic acid contained in a tumor specimen of the subject, and the second library sample is prepared from ribonucleic acid contained in the tumor specimen of the subject, and
the analysis information comprises information on a somatic mutation based on an analysis result of the first sequence data and information on a fusion gene mutation based on an analysis result of the second sequence data.

6. The control method according to claim 5, wherein
the sequence data set further comprises third sequence data corresponding to a third library sample prepared from a non-tumor specimen of the same subject,
the link information is information indicating that the third library sample is prepared from the specimen of the same subject in addition to the first library sample and the second library sample,
the analyzing a first sequence data and a second sequence data comprises analyzing the third sequence data, and
the analysis information further comprises germline mutation information based on an analysis result of the third sequence data in addition to the somatic mutation information based on the analysis result of the first sequence data and the fusion gene mutation information based on the analysis result of the second sequence data.

7. The control method according to claim 4 or 6, wherein
the non-tumor specimen is a blood sample collected from the subject.

8. The control method according to any one of claims 1 to 7, further comprising
receiving analysis request information comprising at least one of case information of the subject, a type of the gene panel test, and first facility information from the first facility via the network.

9. The control method according to any one of claims 1 to 8, the method further comprising
obtaining input information, inputted by a human to a third computer at the first facility, indicating that the first library sample and the second library sample are prepared from the specimen of the same subject, and
comparing the link information and the input information.

10. The control method according to claim 9, further comprising
determining whether the link information and the input information are consistent with each other, and wherein
in response to the link information and the input information being consistent, the analyzing the first sequence data and the second sequence data is executed.

11. The control method according to claim 9 or 10, further comprising
determining whether the link information and the input information are consistent with each other, and
in response to the link information and the input information being inconsistent, notifying error information based on the inconsistency to the first facility.

12. The control method according to any one of claims 1 to 11, further comprising
receiving, with the sequence data set, another sequence data set comprising a plurality of nucleic acid sequence data obtained by the sequencer, corresponding to each of a plurality of library samples comprising a fourth library sample and a fifth library sample prepared from a specimen of another subject.

13. The control method according to claim 12, wherein
the first library sample, the second library sample, the fourth library sample, and the fifth library sample are samples, in which sequences are read by the sequencer in the same sequence run.

14. The control method according to any one of claims 1 to 13, wherein
the receiving the sequence data set and the link information, the analyzing the first sequence data and the second sequence data, and the outputting the analysis information are performed by a computer in a cloud system.

15. The control method according to any one of claims 1 to 14, wherein
the link information is used as sample identification information to identify a library sample or subject identification information to identify a subject from whom a specimen of a library sample is collected.

16. An analysis system that analyzes, at a second facility, nucleic acid sequence data obtained, at a first facility, by a sequencer that reads a nucleic acid sequence, for a gene panel test, comprising:
a first computer configured to
receive, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject, and
send the sequence data set and the link information obtained from the first facility to a second computer; and
the second computer configured to
analyze a first sequence data and a second sequence data corresponding to each of the first library sample and the second library sample linked by the link information, and
output analysis information based on an analysis result of the first sequence data and an analysis result of the second sequence data.

17. An analysis system that analyzes, at a second facility, nucleic acid sequence data obtained, at a first facility, by a sequencer that reads a nucleic acid sequence, for a gene panel test, comprising:
a computer configured to
receive, from the first facility via a network, a sequence data set comprising a plurality of nucleic acid sequence data obtained by the sequencer corresponding to each of a plurality of library samples comprising a first library sample and a second library sample, which are prepared from a specimen of a subject, and link information indicating that the first library sample and the second library sample are prepared from the specimen of the same subject,
analyze a first sequence data and a second sequence data corresponding to each of the first library sample and the second library sample linked by the link information, and
output analysis information based on an analysis result of the first sequence data and an analysis result of the second sequence data.
